# EUROPEAN PATENT SPECIFICATION

(11) **EP 2 260 035 B1**
(45) Date of publication and mention of the grant of the patent: **20.08.2014**
(21) Application number: 09717753.9
(22) Date of filing: 04.03.2009
(51) Int. Cl.: C07D 471/04, C07D 487/04, C07D 487/22, C07D 495/16, C07C 15/38

(54) **POLYCYCLIC ORGANIC COMPOUNDS, POLARIZING ELEMENTS AND METHOD OF PRODUCTION THEREOF**
POLYCYCLISCHE ORGANISCHE VERBINDUNGEN, POLARISIERUNGSELEMENTE UND VERFAHREN ZU DEREN HERSTELLUNG
COMPOSÉS ORGANIQUES POLYCYCLIQUES, ÉLÉMENTS POLARISANTS ET LEUR PROCÉDÉ DE FABRICATION

(30) Priority: 04.03.2008 GB 0804082
(43) Date of publication of application: 15.12.2010
(73) Proprietor: CRYSOPTIX K.K., Tokyo (JP)
(72) Inventor: NOKEL, Alexey, Moscow 119619 (RU); LAZAREV, Pavel I., London Greater London NW3 3SU (GB)
(74) Representative: Williams, Paul Edwin
(86) International application number: PCT/GB2009/050218
(87) International publication number: WO 2009/109781

(56) References cited:
- EP-A1- 1 632 494
- WO-A1-2007/029533
- DE-A1-102005 055 298
- US-A1- 2005 196 550
- DATABASE CAPLUS [Online] CHEMICAL ABSTRACTS SERVICE, COLUMBUS, OHIO, US; 7 October 2007 (2007-10-07), XP002543542 retrieved from STN Database accession no. 2007:1167020 & VAKULIN, I. V. ET AL.: "Quantum-chemical study of formation of complexes of aromatic Schiff bases with transition metals using the UB3LYP/3-21G(d) approximation" BASHKIRSKII KHIMICHESKII ZHURNAL, vol. 14, no. 1, 2007, pages 124-128, Bashkir State University, Ufa; RU ISSN: 0869-8406
- LIANG, B. ET AL.: "HIGH-EFFICIENCY RED PHOSPHORESCENT IRIDIUM DENDRIMERS WITH CHARGE-TRANSPORTING DENDRONS: SYNTHESIS AND ELECTROLUMINESCENT PROPERTIES" ADVANCED FUNCTIONAL MATERIALS, vol. 17, no. 17, 23 November 2007 (2007-11-23), pages 3580-3589, XP001509448 WILEY VCH, WEINHEIM; DE ISSN: 1616-301X
- DATABASE CAPLUS [Online] CHEMICAL ABSTRACTS SERVICE, COLUMBUS, OHIO, US; 13 February 2008 (2008-02-13), XP002543543 retrieved from STN Database accession no. 2008:180123 & KAWABATA, H. ET AL.: "A hybrid DFT study on the mechanism of the hole transfer in dibenzosilole oligomers" IPAP CONFERENCE SERIES 2005, 6 (PROCEEDINGS OF THE INTERNATONAL SYMPOSIUM ON SUPER-FUNCTIONALITY ORGANIC DEVICES, 2004), vol. 6, 2005, pages 42-45, INSTITUTE OF PURE AND APPLIED PHYSICS, TOKYO; JP

## Description

The present invention relates to organic chemistry, in particular, to polycyclic organic compounds, optically anisotropic layers based on these compounds and methods of production thereof. More specifically, the present invention is related to the optical films for liquid crystal displays and in particular to polarizing elements.

Polarizing elements based on polyvinyl alcohol (PVA) are widely used in production of liquid-crystal indicators for screens, watches, calculators, computers, mobile phones etc. The conventional polarizers based on the polyvinyl alcohol films dyed with iodine vapours have high polarization characteristics. However these polarizers have a low mechanical strength and low stability under humid conditions, and for these reasons require special arrangements for protection against mechanical damages or environment, which makes liquid crystal devices more complicated and expensive.

One of the alternative technologies for the polarizing elements is manufacturing of the dye-based thin optical films. The ability of organic dyes to form liquid crystal phases, the methods of making the film from the dye solutions and the optical properties of dye-based thin films are described in J. Lydon, Chromonics, in Handbook of Liquid Crystals, Wiley VCH, Weinheim (1998), Vol. 2B, p. 981-1007.

Linearly polarizing elements are usually dichroic which means a capability of absorbing one of two orthogonal polarized components of an electromagnetic radiation and transmission the other. Due to the random positioning of the molecules of the dichroic material, selective absorption by the individual molecules will cancel each other polarising effect. Thus, it is usually required to orient the molecules to achieve a net linear polarization. Certain molecules, including dichroic dyes, are capable of forming such oriented structures, namely supramolecular structures, in the solutions. The thin films thus obtained are rendered anisotropic either by preliminary mechanical orientation of the substrate surface or by applying external mechanical, electromagnetic, or other orienting actions applied to the liquid crystal layer material on the substrate.

It is known a polarizer which comprises a thin film of the molecularly oriented layer of water-soluble dyes in which sulfo-groups provide water-solubility and which is capable of forming a stable lyotropic liquid crystal phase. A general disadvantage of water soluble organic compounds used in the aforementioned polarizers is that organic layers based on these compounds are characterised by low stability in high humidity conditions and by depolarisation of the transmitted light. Thus there is a need to provide new polarizing materials with good environment stability and mechanical strength. The present invention suggests a decision to the problems mentioned above.

EP1632494 discloses non-peptide vasopressin V1a antagonists and pharmaceutical compositions comprising such, and the use of non-peptide vasopressin V1a antagonists for the treatment of certain physiological disorders, such as primary dysmenorrhoea and Raynaud's disease.

WO2007029533 discloses an organic electroluminescent element, an illuminating device and a display device. An emission wavelength is controlled and a high emission efficiency and long emission life are achieved in disclosed electroluminescent element.

BASHKIRSKII KHIMICHESKII ZHURNAL, vol. 14, no. 1, 2007, pages 124-128, Bashkir State University, Ufa; RU. Discloses a quantum-chemical study of formation of complexes of aromatic Schiff bases with transition metals using the UB3LYP/3-21G(d) approximation. In this article the gas-phase structure and thermo-chemical parameters of Ni²⁺ and Co²⁺ complexes with 2-(aminomethyl)-6-[(phenylimino)methyl]-phenol and its derivatives as polydentate liquids were calculated.

Liang Boet al., Adv. Funct. Mater. 2007,17, 3580-3589 discloses the synthesis of a series of 1-phenylisoquinoline derivatives encapsulated with peripheral arylamines as dendrons using the Ullmann reaction and palladium-catalyzed aromatic carbon-carbon Suzuki-coupling reactions. Red-emitting dendritic iridium complexes (called **G1-1, G1-2,** and G2) are synthesized. The obtained dendrimers are soluble in common organic solvents, and uniform thin films can be spin-coated from such solutions.

Kawabata H. et al., IPAP Conference Series 2005, 6, vol. 6, 2005, pages 42-45 discloses a hybrid DFT (Density Functional Theory) method for the study of the mechanism of the hole transfer in dibenzosilole oligomers. The structures and electronic states of dibenzosilole oligomers were studied by DFT-method to shed light on the mechanism of the hole transport along the chain.

US 2005/196550 discloses a compensator which comprises a birefringent material having a crystal structure formed by at least one polycyclic organic compound with a conjugated π-system. The modifying functional groups providing solubility of the polycyclic organic compounds are selected from the group including -COOH,-SO₃H, PO₃H, NH₂.

DE102005055298 discloses an in plane switching liquid crystal display device comprising first and second polarizing films in which are included poly-vinyl alcohol (PVA).

EP1632494 discloses non-peptide vasopressin V1a antagonists and pharmaceutical compositions comprising such, and the use of non-peptide vasopressin V1a antagonists for the treatment of certain physiological disorders, such as primary dysmenorrhoea and Raynaud's disease.

WO2007029533 discloses an organic electroluminescent element, an illuminating device and a display device. An emission wavelength is controlled and a high emission efficiency and long emission life are achieved in disclosed electroluminescent element.

BASHKIRSKII KHIMICHESKII ZHURNAL, vol. 14, no. 1, 2007, pages 124-128, Bashkir State University, Ufa; RU. Discloses a quantum-chemical study of formation of complexes of aromatic Schiff bases with transition metals using the UB3LYP/3-21G(d) approximation. In this article the gas-phase structure and thermo-chemical parameters of Ni²⁺ and Co²⁺ complexes with 2-(aminomethyl)-6-[(phenylimino)methyl]-phenol and its derivatives as polydentate liquids were calculated.

Liang Boet al., Adv. Funct. Mater. 2007, 17, 3580-3589 discloses the synthesis of a series of 1-phenylisoquinoline derivatives encapsulated with peripheral arylamines as dendrons using the Ullmann reaction and palladium-catalyzed aromatic carbon-carbon Suzuki-coupling reactions. Red-emitting dendritic iridium complexes (called **G1-1, G1-2,** and G2) are synthesized. The obtained dendrimers are soluble in common organic solvents, and uniform thin films can be spin-coated from such solutions.

Kawabata H. et al., IPAP Conference Series 2005, 6, vol. 6, 2005, pages 42-45 discloses a hybrid DFT (Density Functional Theory) method for the study of the mechanism of the hole transfer in dibenzosilole oligomers. The structures and electronic states of dibenzosilole oligomers were studied by DFT-method to shed light on the mechanism of the hole transport along the chain.

US 2005/196550 discloses a compensator which comprises a birefringent material having a crystal structure formed by at least one polycyclic organic compound with a conjugated π-system. The modifying functional groups providing solubility of the polycyclic organic compounds are selected from the group including -COOH,-SO₃H, PO₃H, NH₂.

DE102005055298 discloses an in plane switching liquid crystal display device comprising first and second polarizing films in which are included poly-vinyl alcohol (PVA).

Definitions of various terms used in the description and claims of the present invention are listed below.

The term "partially aromatic" refers to an aromatic conjugated system within a molecule. Examples of aromatic systems are selected from the list but not limited to benzene, naphthalene, coronene etc.

The term "polarizing element" or "polarizer" is used to mean an optical device which in a given state of polarization transmits certain component of light of certain wavelength subrange, and blocks other components. A typical polarizing element consists of an anisotropically absorbing layer, a substrate, and may contain additional layers. The additional layers may comprise a protective layer, an adhesive layer and others - depending on the design and type of the final optical device, for example, a liquid crystal display.

The term "absorbing layer" refers to an optically anisotropic layer obtained from a solution of the disclosed polycyclic organic compound in organic solvent. The layer as used herein refers also to any stack formed of absorbing layers regardless of the number of layers thereof.

The term "visible spectral range" refers to the spectral range of which the lower boundary equals approximately to 400 nm, and upper boundary equals to approximately 700 nm.

A more complete assessment of the present invention and its advantages will be readily achieved as the same becomes better understood by reference to the following detailed description, considered in connection with the accompanying examples and detailed specification, all of which forms a part of the disclosure.

In a first aspect, the present invention provides a polycyclic organic compound of a general structural formula I 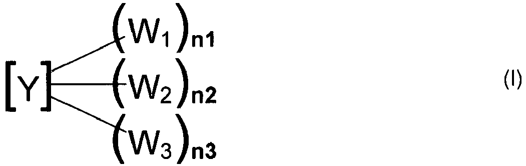 wherein Y is a predominantly planar polycyclic system being at least partially aromatic, W₁, W₂, and W₃ are different groups providing solubility in an organic solvent, and sum (n1+n2+n3) is 1, 2, 3, 4, 5, 6, 7, 8, 9 or 10. The polycyclic organic compound of the present invention forms supramolecules in the organic solvent, and the compound is capable of absorbing electromagnetic radiation in at least one subrange of the visible spectral range. The polycyclic system Y comprises at least one fragment representing a polycyclic aromatic hydrocarbon. In another embodiment, the polycyclic aromatic hydrocarbons are selected from the list comprising acenaphthene, acenaphthylene, acephenanthrylene, aceanthrylene, anthanthrene, benzo[a]coronene, benzo[a]naphthacene, benzo[a]pyrene, benzo[b]chrysene, benzo[b]fluorene, benzo[c]chrysene, benzo[c]phenanthrene, benzo[e]pyrene, benzo[ghi]fluoranthene, benzo[ghi]naphtho[cde]perylene, benzo[ghi]perylene, benzo[j]fluoranthene, benzo[rst]dinaphtho[defg,ijkl]pentaphene, benzo[rst]phenanthro[1,10,9-cde]pentaphene, benz[a]anthracene, benz[e]acephenanthrylene, benz[rst]anthra[cde]pentaphene, biphenylene, chrysene, coronene, dibenzo[b,def]chrysene, dibenzo[bc,ef]coronene, dibenzo[cd,lm]perylene, dibenzo[g,p]chrysene, dibenzo[j,lm]naphtho[ab]perylene, dibenz[a,c]anthracene, dibenz[a,h]anthracene, dibenz[a,j]anthracene, dinaphtho[defg,opqr]pentacene, fluoranthene, fluorene, hexabenzo[a,cd,f,j,lm,o]perylene, naphthacene, naphthalene, naphtho[a]anthracene, naphtho[bcd]perylene, naphtho[d]coronene, pentabenzo[a,cd,f,j,lm]perylene, pentacene, pentaphene, perylene, phenanthrene, phenanthro[3,4-c]phenanthrene, picene, pyranthrene, pyrene, quaterrylene, tetrabenzo[a,cd,f,lm]perylene, terrylene, trinaphthylene, tetranaphthylene, and triphenylene. At least one of the W groups providing solubility in the organic compound is selected from the list comprising linear and branched (C₁-C₃₅)alkyl, (C₂-C₃₅)alkenyl, and (C₂-C₃₅)alkinyl.

In a second aspect, the present invention provides a solution comprising at least one polycyclic organic compound of a general structural formula I 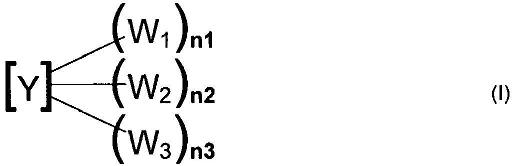 wherein Y is a predominantly planar polycyclic system being at least partially aromatic, W₁, W₂, and W₃ are different groups providing solubility in an organic solvent, and sum (n1+n2+n3) is 1, 2, 3, 4, 5, 6, 7, 8, 9 or 10. Said polycyclic organic compound forms supramolecules in the organic solvent, and the compound is capable of absorbing electromagnetic radiation in at least one subrange of visible spectral range. The solution is capable of forming at least one organic layer ensuring anisotropic absorption of electromagnetic radiation in at least one subrange of the visible spectral range. The polycyclic system Y comprises at least one fragment representing a polycyclic aromatic hydrocarbon. In another embodiment, the polycyclic aromatic hydrocarbons are selected from the list comprising acenaphthene, acenaphthylene, acephenanthrylene, aceanthrylene, anthanthrene, benzo[a]coronene, benzo[a]naphthacene, benzo[a]pyrene, benzo[b]chrysene, benzo[b]fluorene, benzo[c]chrysene, benzo[c]phenanthrene, benzo[e]pyrene, benzo[ghi]fluoranthene, benzo[ghi]naphtho[cde]perylene, benzo[ghi]perylene, benzo[j]fluoranthene, benzo[rst]dinaphtho[defg,ijkl]pentaphene, benzo[rst]phenanthro[1,10,9-cde]pentaphene, benz[a]anthracene, benz[e]acephenanthrylene, benz[rst]anthra[cde]pentaphene, biphenylene, chrysene, coronene, dibenzo[b,def]chrysene, dibenzo[bc,ef]coronene, dibenzo[cd,lm]perylene, dibenzo[g,p]chrysene, dibenzo[j,lm]naphtho[ab]perylene, dibenz[a,c]anthracene, dibenz[a,h]anthracene, dibenz[a,j]anthracene, dinaphtho[defg,opqr]pentacene, fluoranthene, fluorene, hexabenzo[a,cd,f,j,lm,o]perylene, naphthacene, naphthalene, naphtho[a]anthracene, naphtho[bcd]perylene, naphtho[d]coronene, pentabenzo[a,cd,f,j,lm]perylene, pentacene, pentaphene, perylene, phenanthrene, phenanthro[3,4-c]phenanthrene, picene, pyranthrene, pyrene, quaterrylene, tetrabenzo[a,cd,f,lm]perylene, terrylene, trinaphthylene, tetranaphthylene, and triphenylene. At least one of the W groups providing solubility in the organic compound is selected from the list comprising linear and branched (C₁-C₃₅)alkyl, (C₂-C₃₅)alkenyl, and (C₂-C₃₅)alkinyl.

In a third aspect, the present invention provides a polarizing element which comprises at least one organic layer which is capable of anisotropic absorption of the electromagnetic radiation in at least one subrange of the visible spectral range. The organic layer comprises at least one polycyclic organic compound of a general structural formula I 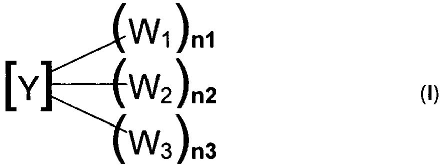 wherein Y is a predominantly planar polycyclic system being at least partially aromatic, W₁, W₂, and W₃ are different groups providing solubility in an organic solvent, and sum (n1+n2+n3) is 1, 2, 3, 4, 5, 6, 7, 8, 9 or 10. The polycyclic system Y comprises at least one fragment representing a polycyclic aromatic hydrocarbon. In another embodiment, the polycyclic aromatic hydrocarbons are selected from the list comprising acenaphthene, acenaphthylene, acephenanthrylene, aceanthrylene, anthanthrene, benzo[a]coronene, benzo[a]naphthacene, benzo[a]pyrene, benzo[b]chrysene, benzo[b]fluorene, benzo[c]chrysene, benzo[c]phenanthrene, benzo[e]pyrene, benzo[ghi]fluoranthene, benzo[ghi]naphtho[cde]perylene, benzo[ghi]perylene, benzo[j]fluoranthene, .benzo[rst]dinaphtho[defg,ijkl]pentaphene, benzo[rst]phenanthro[1,10,9-cde]pentaphene, benz[a]anthracene, benz[e]acephenanthrylene, benz[rst]anthra[cde]pentaphene, biphenylene, chrysene, coronene, dibenzo[b,def]chrysene, dibenzo[bc,ef]coronene, dibenzo[cd,lm]perylene, dibenzo[g,p]chrysene, dibenzo[j,lm]naphtho[ab]perylene, dibenz[a,c]anthracene, dibenz[a,h]anthracene, dibenz[a,j]anthracene, dinaphtho[defg,opqr]pentacene, fluoranthene, fluorene, hexabenzo[a,cd,f,j,lm,o]perylene, naphthacene, naphthalene, naphtho[a]anthracene, naphtho[bcd]perylene, naphtho[d]coronene, pentabenzo[a,cd,f,j,lm]perylene, pentacene, pentaphene, perylene, phenanthrene, phenanthro[3,4-c]phenanthrene, picene, pyranthrene, pyrene, quaterrylene, tetrabenzo[a,cd,f,lm]perylene, terrylene, trinaphthylene, tetranaphthylene, and triphenylene. At least one of the **W** groups providing solubility in the organic compound is selected from the list comprising linear and branched (C₁-C₃₅)alkyl, (C₂-C₃₅)alkenyl, and (C₂-C₃₅)alkinyl.

In a fourth aspect, the present invention provides a method of forming a polarizing element. The disclosed method comprises the steps of:
a) preparation of a solution of a polycyclic organic compound of the general structural formula I in an organic solvent 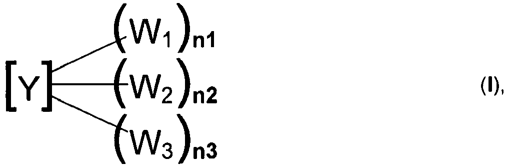 wherein Y is a predominantly planar polycyclic system being at least partially aromatic, W₁, W₂, and W₃ are different groups providing solubility in an organic solvent, and sum (n1+n2+n3) is 1, 2, 3, 4, 5, 6, 7, 8, 9 or 8; the polycyclic organic compound is capable of forming supramolecules in the solution, and said compound is capable of absorbing electromagnetic radiation in at least one subrange of the visible spectral range;
b) deposition of a layer of the solution on a substrate; and
c) drying with formation of an optically anisotropic layer.

The polycyclic system Y comprises at least one fragment representing a polycyclic aromatic hydrocarbon. In another embodiment, the polycyclic aromatic hydrocarbons are selected from the list comprising acenaphthene, acenaphthylene, acephenanthrylene, aceanthrylene, anthanthrene, benzo[a]coronene, benzo[a]naphthacene, benzo[a]pyrene, benzo[b]chrysene, benzo[b]fluorene, benzo[c]chrysene, benzo[c]phenanthrene, benzo[e]pyrene, benzo[ghi]fluoranthene, benzo[ghi]naphtho[cde]perylene, benzo[ghi]perylene, benzo[j]fluoranthene, benzo[rst]dinaphtho[defg,ijkt]pentaphene, benzo[rst]phenanthro[1,10,9-cde]pentaphene, benz[a]anthracene, benz[e]acephenanthrylene, benz[rst]anthra[cde]pentaphene, biphenylene, chrysene, coronene, dibenzo[b,def]chrysene, dibenzo[bc,ef]coronene, dibenzo[cd,lm]perylene, dibenzo[g,p]chrysene, dibenzo[j,lm]naphtho[ab]perylene, dibenz[a,c]anthracene, dibenz[a,h]anthracene, dibenz[a,j]anthracene, dinaphtho[defg,opqr]pentacene, fluoranthene, fluorene, hexabenzo[a,cd,f,j,lm,o]perylene, naphthacene, naphthalene, naphtho[a]anthracene, naphtho[bcd]perylene, naphtho[d]coronene, pentabenzo[a,cd,f,j,lm]perylene, pentacene, pentaphene, perylene, phenanthrene, phenanthro[3,4-c]phenanthrene, picene, pyranthrene, pyrene, quaterrylene, tetrabenzo[a,cd,f,lm]perylene, terrylene, trinaphthylene, tetranaphthylene, and triphenylene. At least one of the **W** groups providing solubility in the organic compound is selected from the list comprising linear and branched (C₁-C₃₅)alkyl, (C₂-C₃₅)alkenyl, and (C₂-C₃₅)alkinyl.

In the disclosed polycyclic organic compound the polycyclic system Y may contain a conjugated system of double and/or triple bonds, and π-π*-transitions in molecules with conjugated double and triple bonds ensure the absorption of the electromagnetic radiation in the visible spectral range.

In another embodiment of the disclosed polycyclic organic compound, the polycyclic system Y is heterocyclic. In still another embodiment the heteroatoms of the heterocyclic system are selected from the list comprising N, O and S. In still another embodiment of the disclosed polycyclic organic compound, the polycyclic system Y comprises at least one fragment selected from the list comprising furan, oxirane, 4*H*-pyran, 2*H*-chromene, benzo[*b*]furan, 2*H*-pyran, thiophene, benzo[b]thiophene, parathiazine, pyrrole, pyrrolidine, pyrazole, imidazole, imidazoline, imidazolidine, pyrazolidine, pyrimidine, pyridine, piperazine, piperidine, pyrazine, indole, purine, benzimidazole, quinoline, phenothiazine, morpholine, thiaziole, thiadiazole, and oxazole.

In yet another embodiment, the polycyclic system **Y** comprises fragments selected from the list comprising perylene, tetrapyrrolic macrocycles, coronene and pyrazine, and having general structural formulas selected from structures 1-46 and shown in the Table 1.

**Table 1. Examples of polycyclic systems Y with perylene, tetrapyrrolic macrocycles, coronene and pyrazine fragments**

| | |
|---|---|
| 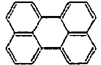 | 1 |
| 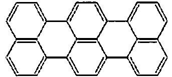 | 2 |
| 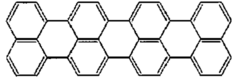 | 3 |
| 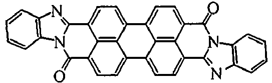 | 4 |
| 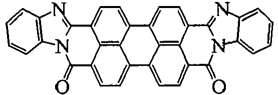 | 5 |
| 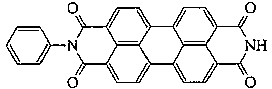 | 6 |
| 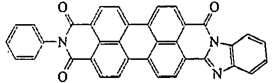 | 7 |
| 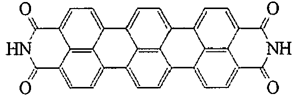 | 8 |
| 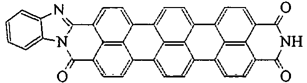 | 9 |
| 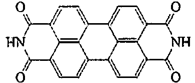 | 10 |
| 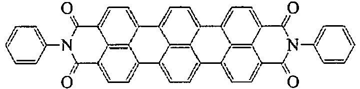 | 11 |
| 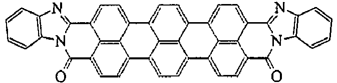 | 12 |
| 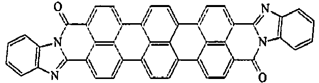 | 13 |
| 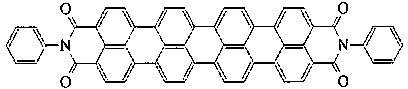 | 14 |
| 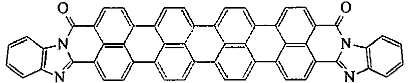 | 15 |
| 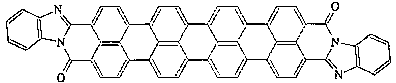 | 16 |
| 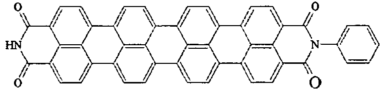 | 17 |
| 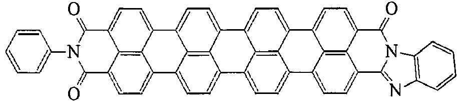 | 18 |
| 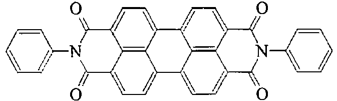 | 19 |
| 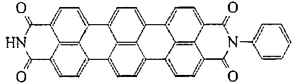 | 20 |
| 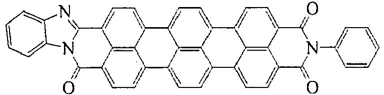 | 21 |
| 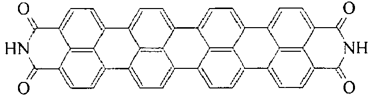 | 22 |
| 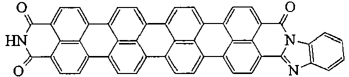 | 23 |
| 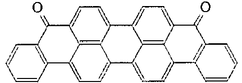 | 24 |
| 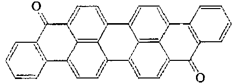 | 25 |
| 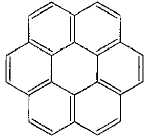 | 26 |
| 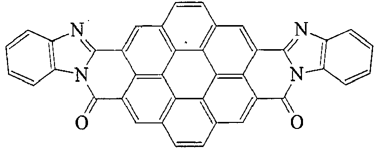 | 27 |
| 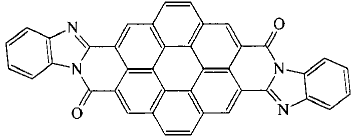 | 28 |
| 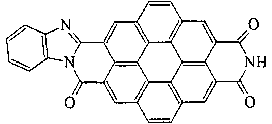 | 29 |
| 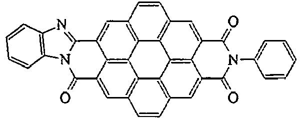 | 30 |
| 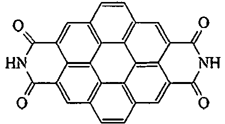 | 31 |
| 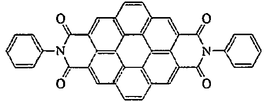 | 32 |
| 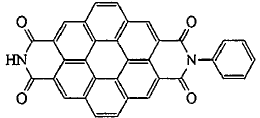 | 33 |
| 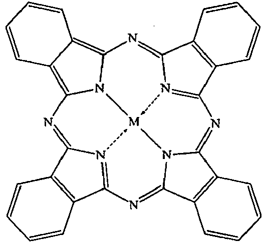 | 34 |
| 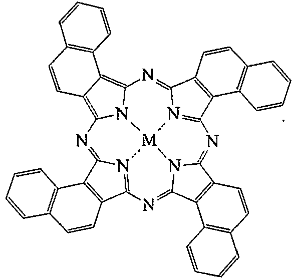 | 35 |
| 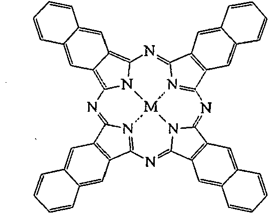 | 36 |
| 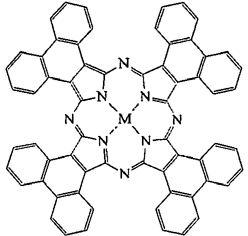 | 37 |
| 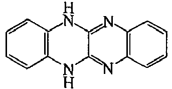 | 38 |
| 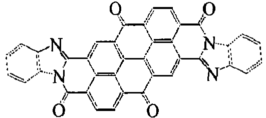 | 39 |
| 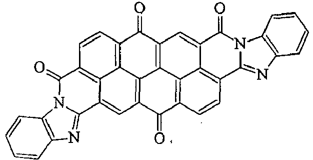 | 40 |
| 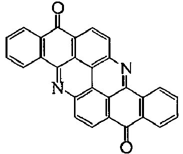 | 41 |
| 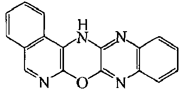 | 42 |
| 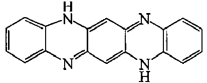 | 44 |
| 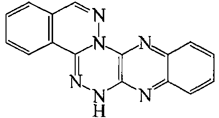 | 45 |
| 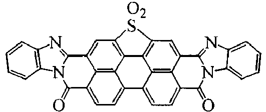 | 46 |

wherein M is selected from the list comprising 2H, Cu, Zn, Co, Fe and Pt.

The **W** groups may be connected with the polycyclic system **Y** via at least one covalent bond. Alkyl groups may form a cycle by connecting to the polycyclic system **Y** via at least two covalent bonds. The hydrophobic interaction between alkyl chains improves solubility by forming supramolecules, and the intermolecular π-π-interactions of unsaturated bonds may play substantial role to ensure the formation of supramolecules in solutions of organic solvents. Hereinafter the term "supramolecules" comprises molecular aggregations in the solution, and the types of supramolecules comprise rod-like, lamellar supramolecules and other types known by those skilled in the art.

In another embodiment of the disclosed polycyclic organic compound, at least one of the **W** groups is connected with the polycyclic system Y via a bridging group **A.** In yet another embodiment, the bridging group **A** is selected from the list, comprising -C(O)-, -C(O)O-, -C(O)-NH-, -(SO₂)NH-, -O-, -CH₂O- -NH-, >N-, and any combination thereof.

In one embodiment of the present invention, the polycyclic organic compound is selected from the list comprising **diimides I.1, I.2, I.3,** and **I.4** shown in Table 2, wherein 1.1 is perylene diimides and **I.2-I.4** are coronene diimides.

**Table 2. Examples of the polycyclic organic compounds of the invention: diimides**

| |
|---|
| 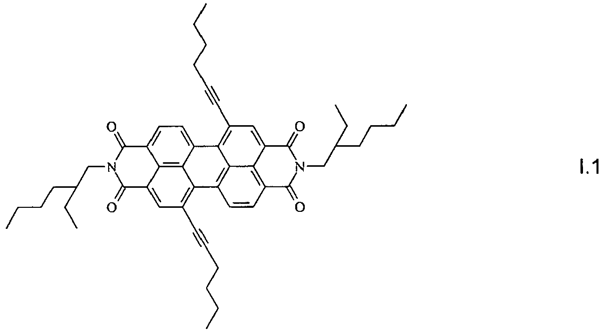 |
| N,N'-di(2-ethylhexyl)-1,7-di(hex-1-ynyl)perylene-3,4:9,10-tetracarboxydiimide |
| 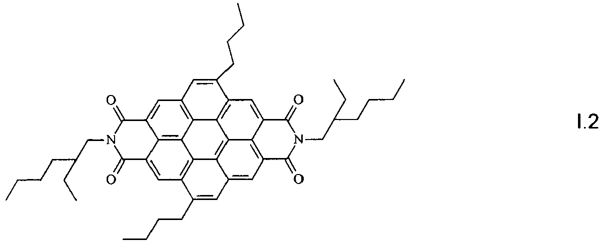 |
| N,N'-di(2-ethylhexyl)-5,11-dibutylcoronene-2,3:8,9-tetracarboxydiimide |
| 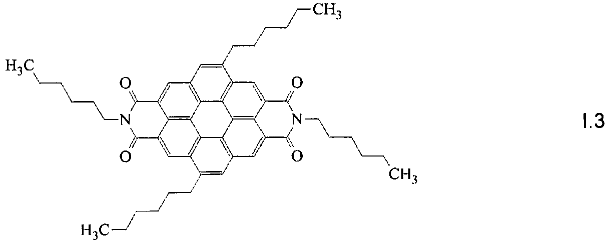 |
| N,N'-dihexyl-5,11-dihexylcoronene-2,3:8,9-tetracarboxydiimide |
| 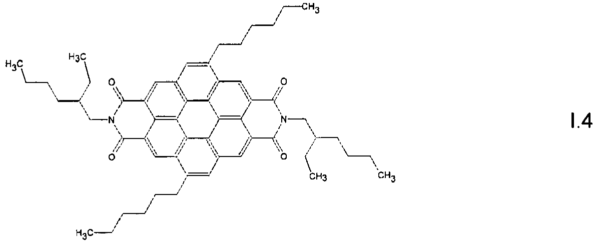 |
| N,N'-di(2-ethylhexyl)-5,11-dihexylcoronene-2,3:8,9-tetracarboxydiimide |

In one embodiment of the disclosed polycyclic organic compound, said polycyclic systems may be capable of forming rod-like supramolecules via π-π-interaction. In another embodiment of the disclosed polycyclic organic compound, the rod-like supramolecules have interplanar spacing between the polycyclic systems in the range of approximately 3.1-3.7 A. In still another embodiment of the present invention, said compound is photochromic. In yet another embodiment of the present invention, the polycyclic organic compound is further capable of absorbing electromagnetic radiation in at least one subrange of the UV spectral range.

The present invention also provides the solution as disclosed hereinabove. In one embodiment of the present invention, the solution comprises a mix of at least two polycyclic organic compounds of the general structural formula I, wherein said polycyclic organic compounds are capable of absorbing of electromagnetic radiation in at least two subranges of the visible spectral range.

In another embodiment of the disclosed solution, the polycyclic system **Y** is heterocyclic. The heteroatoms in said polycyclic system are selected from the list comprising N, O and S. In still another embodiment of the invention, the polycyclic system Y comprises at least one fragment selected from the list comprising furan, oxirane, 4*H*-pyran, 2*H*-chromene, benzo[*b*]furan, 2*H*-pyran, thiophene, benzo[b]thiophene, parathiazine, pyrrole, pyrrolidine, pyrazole, imidazole, imidazoline, imidazolidine, pyrazolidine, pyrimidine, pyridine, piperazine, piperidine, pyrazine, indole, purine, benzimidazole, quinoline, phenothiazine, morpholine, thiaziole, thiadiazole, and oxazole.

In yet another embodiment of the disclosed solution, the polycyclic system **Y** comprises fragments selected from the list comprising perylene, tetrapyrrolic macrocycles, coronene and pyrazine, and having general structural formula selected from the structures 1 to 46 as shown in Table 1.

The W-groups may be connected with the polycyclic system Y via at least one covalent bond. The alkyl groups may form a cycle by connecting to the polycyclic system Y via at least two covalent bonds. The hydrophobic interaction between alkyl chains improves solubility by forming supramolecules, and the intermolecular π-π-interactions of unsaturated bonds may play substantial role to ensure the formation of supramolecules in solutions of organic solvents.

In another embodiment of the disclosed solution, at least one of the **W** groups providing solubility is connected with the polycyclic system **Y** via a bridging group **A.** In yet another embodiment, the bridging group **A** is selected from the list, comprising -C(O)-, -C(O)O-, -C(O)-NH-, -(SO₂)NH-, -O-, -CH₂O-, -NH-, >N-, and any combination thereof.

In yet another embodiment of the disclosed solution, the polycyclic compound of the present invention is selected from the list comprising the compounds **1.1, I.2, I.3** and **I.4** shown in Table 2.

In yet another embodiment of the disclosed solution, the organic solvent is selected from the list comprising ketones, carboxylic acids, hydrocarbons, cyclohydrocarbons, chlorohydrocarbons, alcohols, ethers, esters, and any combination thereof. In the preferred embodiment of the disclosed solution, the organic solvent is selected from the list comprising acetone, xylene, toluene, ethanol, methylcyclohexane, ethyl acetate, diethyl ether, octane, chloroform, methylenechloride, dichloroethane, trichloroethene, tetrachloroethene, carbon tetrachloride, 1,4-dioxane, tetrahydrofuran, pyridine, triethylamine, nitromethane, acetonitrile, dimethylformamide, dimethulsulfoxide, and any combination thereof.

In still another embodiment of the present invention, the disclosed solution is a lyotropic liquid crystal solution. In yet another embodiment of the present invention, the solution is an isotropic solution.

In one embodiment of the disclosed solution, the supramolecules are formed by interaction of at least two different compounds of said formula **I.** In another embodiment of the disclosed solution, the supramolecules are formed by interaction of the same compounds of the general structural formula **I.**

In another embodiment of the invention, the solution further comprises additives, such as surfactants and/or plasticizers which are soluble in the organic solvents. The additives and/or plasticizers are chosen from the compounds which do not damage the alignment of the solution.

The present invention also provides the polarizing element as disclosed hereinabove. In one embodiment of the disclosed polarizing element, said layer comprises two or more said polycyclic compounds of the general structural formula **I,** ensuring absorption of electromagnetic radiation in at least two different predetermined wavelength subranges of the visible spectral range. In another embodiment of the invention, the polarizing element comprises two or more organic layers, wherein each of said layers comprises different polycyclic compounds of the general structural formula I, ensuring the absorption of electromagnetic radiation in at least two predetermined wavelength subranges of the visible spectral range. In this embodiment the polarizing element may comprise two organic layers. One layer may comprise one type of polycyclic compound of the general structural formula I, ensuring the absorption of electromagnetic radiation in one predetermined wavelength subrange of the visible spectral range. Another layer may comprise another type of polycyclic compound of the general structural formula **I,** ensuring the absorption of electromagnetic radiation in another predetermined wavelength subrange of the visible spectral range.

In another embodiment of the polarizing element, the polycyclic system **Y** is heterocyclic. Heteroatoms of said polycyclic system are selected from the list comprising N, O and S. In still another embodiment of the polarizing element, the polycyclic system **Y** comprises at least one fragment selected from the list comprising furan, oxirane, 4*H*-pyran, 2*H*-chromene, benzo[*b*]furan, 2H-pyran, thiophene, benzo[*b*]thiophene, parathiazine, pyrrole, pyrrolidine, pyrazole, imidazole, imidazoline, imidazolidine, pyrazolidine, pyrimidine, pyridine, piperazine, piperidine, pyrazine, indole, purine, benzimidazole, quinoline, phenothiazine, morpholine, thiaziole, thiadiazole, and oxazole.

In yet another embodiment of the disclosed polarizing element, the polycyclic system Y comprises fragments selected from the list comprising perylene, tetrapyrrolic macrocycles, and pyrazine, and having the general structural formula selected from the structures 1 to 49 as shown in the Table 1.

In another embodiment of the disclosed polarizing element, at At least one of the **W** groups providing solubility is may be connected with the polycyclic system **Y** via a bridging group **A.** In yet another embodiment, the bridging group **A** is selected from the list, comprising -C(O)-, -C(O)O-, - C(O)-NH-, -(SO₂)NH-, -O-, -CH₂O-, -NH-, >N-, and any combination thereof.

In yet another embodiment of the disclosed polarizing element, the polycyclic compound of the present invention is selected from the list comprising the compounds **1.1, 1.2, 1.3** and **1.4** shown in Table 2.

The organic solvent may be selected from the list comprising ketones, carboxylic acids, hydrocarbons, cyclohydrocarbons, chlorohydrocarbons, alcohols, ethers, esters, and any combination thereof. In the preferred embodiment of the disclosed solution, the organic solvent is selected from the list comprising comprising acetone, xylene, toluene, ethanol, methylcyclohexane, ethyl acetate, diethyl ether, octane, chloroform, methylenechloride, dichloroethane, trichloroethene, tetrachloroethene, carbon tetrachloride, 1,4-dioxane, tetrahydrofuran, pyridine, triethylamine, nitromethane, acetonitrile, dimethylformamide, dimethulsulfoxide, and any combination thereof. In another embodiment of the disclosed polarizing element, the organic compound is photochromic.

In one embodiment of the present invention, the disclosed polarizing element further comprises a substrate. In another embodiment of the disclosed polarizing element, the substrate is transparent for electromagnetic radiation in the visible spectral range. In still another embodiment of the disclosed polarizing element, the substrate may be made of polymer. In yet another embodiment the substrate may be made of glass. For the reflective LCDs the substrate may be made of foil having specular or diffuse reflecting surface. In one embodiment the polarizing element further comprises a transparent adhesive layer applied on top of the organic layer. In yet another embodiment the polarizing element further comprises a protective coating applied on the adhesive transparent layer.

In one embodiment of the polarizing element, the optically anisotropic organic layer is at least partially crystalline.

The present invention also provides a method for producing the polarizing element, as disclosed hereinabove. In one embodiment of the present invention, the disclosed method further comprises an application of an external orienting action onto the layer of the solution in order to provide dominant orientation of supramolecules. The orienting action may take place after the step b) of the deposition of the layer of the solution. In another embodiment it may take place simultaneously with the step b). The orienting action may be selected from the list comprising external mechanical, electromagnetic, other orienting actions known from the art and any combinations thereof.

In another embodiment of the disclosed method, the polycyclic system Y is heterocyclic. In still another embodiment of the disclosed method, at least one heteroatom of the heterocyclic system Y is selected from the list comprising N, O and S. In yet another embodiment, the polycyclic system Y comprises at least one fragment selected from the list comprising furan, oxirane, 4*H*-pyran, 2*H-*chromene, benzo[*b*]furan, 2*H*-pyran, thiophene, benzo[*b*]thiophene, parathiazine, pyrrole, pyrrolidine, pyrazole, imidazole, imidazoline, imidazolidine, pyrazolidine, pyrimidine, pyridine, piperazine, piperidine, pyrazine, indole, purine, benzimidazole, quinoline, phenothiazine, morpholine, thiazole, thiadiazole, and oxazole

In yet another embodiment of the disclosed method, the polycyclic system **Y** comprises fragments selected from the list comprising perylene, tetrapyrrolic macrocycles, coronene and pyrazine, and having a general structural formula selected from structures 1 to 46 as shown in Table 1.

In one embodiment of disclosed method, at least one of the **W** groups providing solubility is connected with the polycyclic system Y via a bridging group **A.** In yet another embodiment, the bridging group **A** is selected from the list, comprising -C(O)-, -C(O)O-, -C(O)-NH-, -(SO₂)NH-, -O-, -CH₂O-, -NH-, >N-, and any combination thereof.

In yet another embodiment of the disclosed method, the polycyclic compound of the present invention is selected from the list comprising compounds **I.1, I.2, I.3** and **I.4** shown in Table 2.

The organic solvent is selected from the list comprising ketones, carboxylic acids, hydrocarbons, cyclohydrocarbons, chlorohydrocarbons, alcohols, ethers, esters, and any combination thereof. In the preferred embodiment of the disclosed method, the organic solvent is selected from the list comprising comprising acetone, xylene, toluene, ethanol, methylcyclohexane, ethyl acetate, diethyl ether, octane, chloroform, methylenechloride, dichloroethane, trichloroethene, tetrachloroethene, carbon tetrachloride, 1,4-dioxane, tetrahydrofuran, pyridine, triethylamine, nitromethane, acetonitrile, dimethylformamide, dimethulsulfoxide, and any combination thereof..

The substrate may be made of polymer or glass. The substrate may be made of foil which might have a specular or diffuse reflecting surface.

In another embodiment of the present invention, the method further comprises the step of removing the substrate by one of the methods chosen from the list comprising wet chemical etching, dry chemical etching, plasma etching, laser etching, and grinding. The sequence of the steps of preparation of a solution of a polycyclic organic compound of the general structural formula I, deposition of a layer of the solution on a substrate, and the drying may be repeated two or more times and each consequent optically anisotropic layer is formed using the organic solution, this solution being either the same or different from that used in the previous cycle and having the absorption of the electromagnetic radiation in at least one predetermined wavelength subrange of the visible spectral range.

The general description of the present invention having been made, a further understanding can be obtained by reference to the specific preferred embodiments, which are given herein only for the purpose of illustration and are not intended to limit the scope of the appended claims provided below, and upon reference to the drawings, in which:
Figure 1 shows the polarizing element according to the present invention;
Figure 2 shows coronene derivative used in an embodiment for preparation of the polarizing element;
Figure 3 shows a typical absorption spectrum of the polarizing element according to an embodiment of the present invention;
Figure 4 shows a dichroic ratio of the polarizing element according to one embodiment of the present invention; and

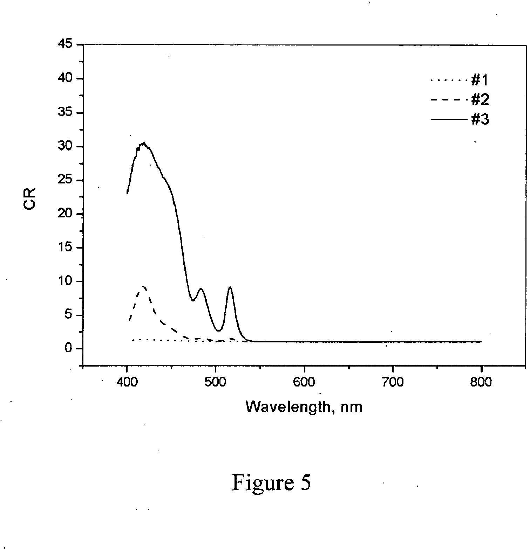

Figure 5 shows a contrast ratio of the polarizing element according to one embodiment of the present invention.

Other objects and advantages of the present invention will become apparent upon reading detailed description of the examples and the appended claims provided below. The following examples are detailed descriptions of methods of preparation and use of certain compounds of the present invention. Although there is only one detailed synthetic example illustrating each polycyclic system Y (Table 1), it should be understood that the scope of the invention in not limited to these specific structures as many other variations with different **W**-groups can be readily obtained using the provided procedures. The examples are presented to illustrate the embodiments of the invention and are not intended as a restriction on the scope of the invention.

The following examples describing detailed preparation of the polarizing elements are included for the purpose of illustration and the person skilled in the art can obtain the polarizing elements with any other compound of the present invention. In the following examples, all percentages are weight percentages and all temperatures are in centigrade.

### EXAMPLE 1

Example 1 describes preparation of the N,N'-di(2-ethylhexyl)-1,7-di(hex-1-ynyl)perylene-3,4:9,10-tetracarboxydiimide **(I.1;** Table 2).

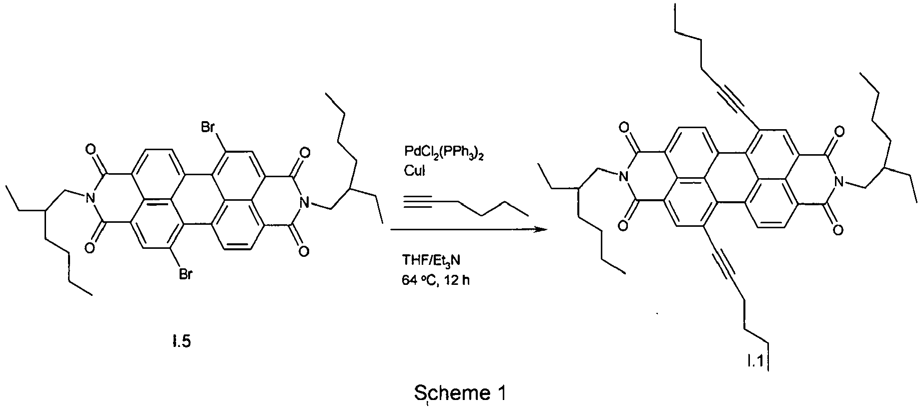

Synthetic procedure is presented by Scheme 1. The perylene diimide **I.5** is shown as an initial compound in Scheme 1. The perylene diimides **I.5** (4.2 g, 5.4 mmol) were placed into a three-neck 0.6 L flask equipped with a thermometer, argon inlet tube, air condenser and bubble counter.

The apparatus was evacuated and filled with argon several times. Anhydrous degassed tetrahydrofuran (400 mL) was added to the reaction vessel, followed by triethylamine (6.6 g, 9.0 mL, 66 mmol), bis(triphenylphosphine)palladium(II) chloride, (0.38 g, 0.54 mmol), copper (I) iodide (0.20 g, 1.1 mmol), and triphenylphosphine (0.14 g, 0.54 mmol). The resultant suspension was evacuated until slight boiling occurred and then the reaction vessel was filled with argon. The last step was repeated three times using vacuum of a membrane pump. Then 1-hexyne was added (2.2 g, 3.18 mL, 15 mmol), and the reaction mixture was heated with simultaneous stirring until the mild boiling of the reaction mixture (64°C). Heating was continued for 14 hours. Thin layer chromotography (TLC, Merck, silica gel 60 UV 254, eluent chloroform-ethylacetate 100:1) does not show any presence of the initial perylene diimide **I.5.**

The resultant dark violet solution was separated from a small amount of a precipitate by filtration, evaporated to half of initial volume under reduced pressure (rotary evaporator) and poured into the mixture of 70 mL of hydrochloric acid (36%) with ice and water (200 mL). The resultant mixture was extracted with dichloromethane (500 mL), organic phase was washed with water (3x200 mL), and filtered through silica gel (200 mL). The filtrate was then filtered through a paper filter, and evaporated on a rotary evaporator to 100 mL volume. Isopropanol was added (250 mL), and total volume was decreased to 145 mL (114-117 g). Then methanol was added drop-wise with a hand stirring (80 mL). The separated dark violet crystals were filtered off. The yield was 4 to 5 g. The crystals were then dissolved in dichloromethane (100 mL), isopropanol was added (200 mL), and total volume of the solution was decreased to 145 mL. Then methanol (60 mL) was added dropwise with stirring. The separated dark violet crystals were filtered off. The formed crystals were washed with isopropanol-methanol mixture with 20 mL and 3 mL of the parts respectively, and dried for 3 hours at 40° C in vacuum.

The yield of the preparation of N,N'-di(2-ethylhexyl)-1,7-di(hex-1-ynyl)perylene-3,4:9,10-tetracarboxydiimide **I.1** was 3.1 g (74%).

### EXAMPLE 2

Example 2 describes preparation of the compound N,N'-di(2-ethylhexyl)-5,11-dibutylcoronene-2,3:8,9-tetracarboxydiimide **(I.2,** Table 2).

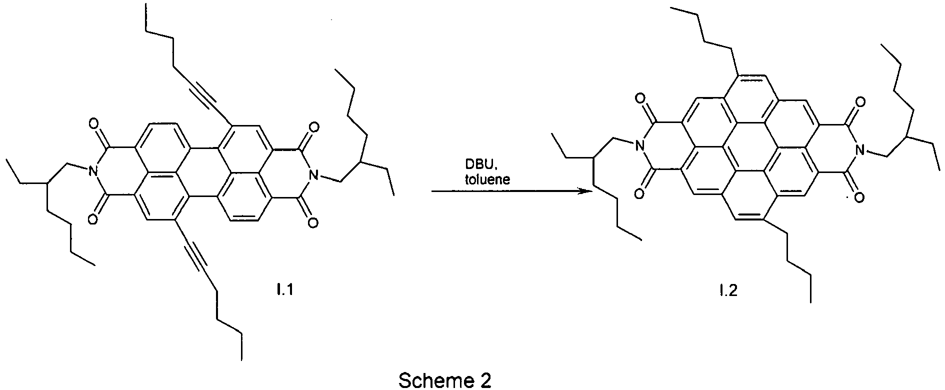

Synthetic procedure is presented by Scheme 2. A mixture of 1.5g N,N'-di(2-ethylhexyl)-1,7-di(hex-1-ynyl)perylene-3,4:9,10-tetracarboxydiimide (1.1) and 3.0 mL of 1,8-diazabicyclo[5.4.0]undec-7-ene (DBU) in 100 mL of toluene was refluxed for 20 hours under argon atmosphere. After cooling the resultant mixture was poured into 6N hydrochloric acid (300 mL) and then extracted with methylene chloride (500 mL). Organic phase was separated and dried over magnesium sulfate, and then solution was filtered out, concentrated and crystallized in hot methanol to produce a crude N,N'-di(2-ethylhexyl)-5,11-dibutylcoronene-2,3:8,9-tetracarboxydiimide (1.3 g). The product was purified on a column with an eluent made of silica gel and chloroform-petroleum ether (3:1).

### EXAMPLE 3

Example 3 describes preparation of N,N'-(1-undecyl)dodecyl-5,11-dihexylcoronene-2,3:8,9-tetracarboxydiimide, the predominantly planar polycyclic system of which is presented in Table 1, structural formula 31. This example is also representative for synthesis of compounds possessing polycyclic aromatic systems with structural formulas 26, 32 and 33, depicted in Table 1. The synthetic procedure is shown in Scheme 3 and comprises six steps.

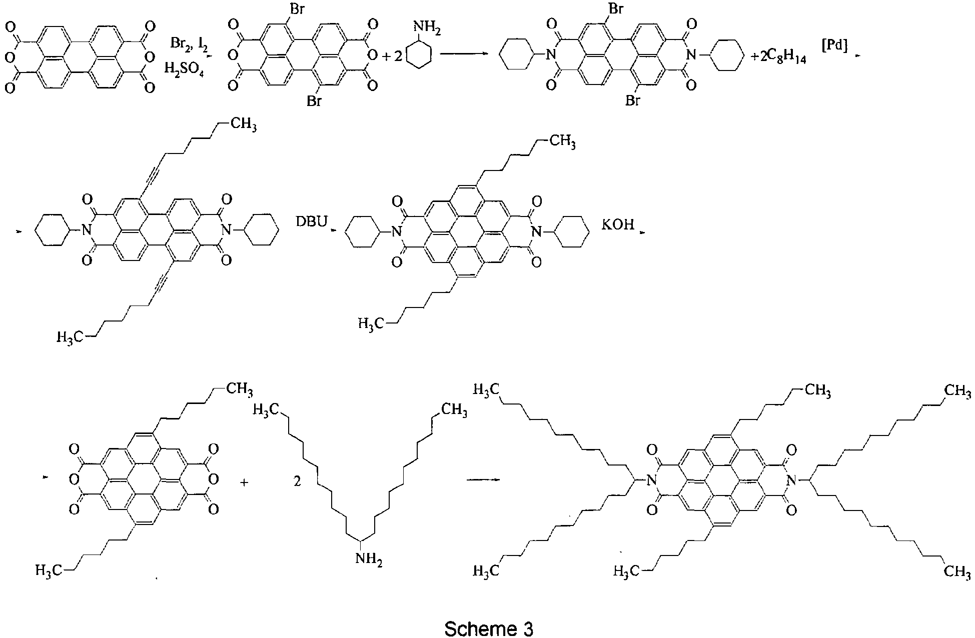

Commercially available Perylene-3,4:9,10-tetracarboxylic dianhydride (100.0 g, 0.255 mol) was brominated with mixture of bromine (29 mL) and Iodine (2.38 g) in 100% sulfuric acid (845 mL) at.~ 85° C. The yield of 1,7-dibromoperylene-3,4:9,10-tetracarboxylic dianhydride was 90 g (64%).

Analysis: calculated: C₂₄H₆Br₂O₆, C 52.40, H 1.10, Br 29.05, O 17.45 %; found: C 52.29, H, 1.07, Br 28, 79 %. Absorption spectrum (9.82x10⁻⁵ M solution in 93% sulfuric acid): 405 (9572), 516 (27892), 553 (37769).

N,N'-Dicyclohexyl-1,7-dibromoperylene-3,4:9,10-tetracarboxydiimide was synthesized by reaction of 1,7-dibromoperylene-3,4:9,10-tetracarboxylic dianhydride (30.0 g) with cyclohexylamine (18.6 mL) in N-methylpyrrolidone (390 mL) at ∼85 °C. The yield of N,N'-dicyclohexyl-1,7-dibromoperylene-3,4:9,10-tetracarboxydiimide was 30 g (77%).

N,N'-Dicyclohexyl-1,7-di(oct-1-ynyl)perylene-3,4:9,10-tetracarboxydiimide by Sonogashira reaction: N,N'-dicyclohexyl-1,7-dibromperylene-3,4:9,10-tetracarboxydiimide (24.7 g) and octyne-1 (15.2 g) in the presence of bis(triphenylphosphine)palladium(II) chloride (2.42 g), triphenylphospine (0.9 g),and copper(l) iodide (0.66 g). The yield of N,N'-dicyclohexyl-1,7-di(oct-1-ynyl)perylene-3,4:9,10-tetracarboxydiimide was 15.7 g (60 %).

N,N'-Dicyclohexyl-5,11-dihexylcoronene-2,3:8,9-tetracarboxydiimide was synthesized by heating of N,N'-dicyclohexyl-1,7-di(oct-1-ynyl)perylene-3,4:9,10-tetracarboxydiimide (7.7 g) in toluene (400 mL) in the presence of 1,8-Diazabicyclo[5.4.0]undec-7-ene (0.6 ml) at 100-110° C for 20 hours.

5,11-dihexylcoronene-2,3:8,9-tetracarboxylic dianhydride was prepared by hydrolysis of N,N'-dicyclohexyl-5,11-dihexylcoronene-2,3:8,9-tetracarboxydiimide (6.4 g, 8.3 mmol) with Potassium hydroxide (7.0 g, 85%) in the mixture of tert-butanol (400 mL) and water (0.4 mL) at 85-90°C. The yield of 5,11-dihexylcoronene-2,3:8,9-tetracarboxylic dianhydride was 4.2 g (83%).

N,N'-(1-undecyl)dodecyl-5,11-dihexylcoronene-2,3:8,9-tetracarboxydiimide by the reaction of 5,11-di(hexyl)coronene-2,3:8,9-tetracarboxylic dianhydride with 12-tricosanamine. 5,11-di(hexyl)coronene-2,3:8,9-tetracarboxylic dianhydride (3.44 g), 12-tricosanamine (7.38 g), benzoic acid (45 mg) and 3-chlorophenol (15 mL) was evacuated and saturated with argon two times at room temperature and 2 times at 100°C. The reaction mixture was agitated at ∼140°C for 1 hour and 160-165°C for 20 hours in a flow of argon. After that the reaction mixture was agitated at ∼100°C and was vacuumed at 10 mm Hg for half an hour. Then apparatus was filled with argon once again and heating was continued for the next 24 hours. The yield of preparation of N,N'-(1-undecyl)dodecyl-5,11-dihexylcoronene-2,3:8,9-tetracarboxydiimide was 5.0 g (70%).

### EXAMPLE 4

Example 4 describes preparation of zinc-4,4',4",4"'-tetracarboxyphthalocyanine, the predominantly planar polycyclic system of which is presented in Table 1, structural formula 34. This example is also representative for synthesis of compounds possessing polycyclic aromatic systems with structural formulas 35, 36 and 37, depicted in Table 1. The synthetic procedure is shown in Scheme 4 and comprises two steps performed in one pot.

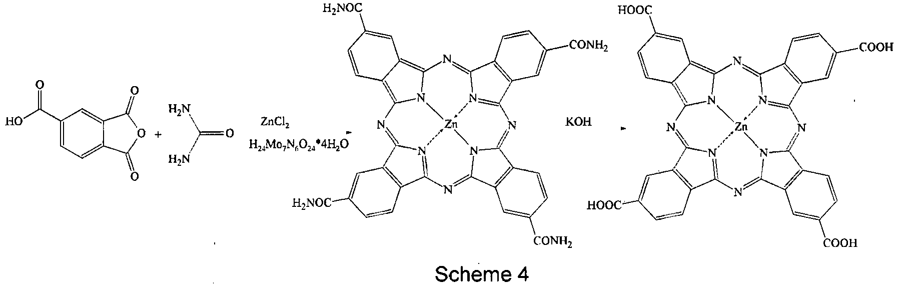

### A. Synthesis of zinc-4,4',4",4'''-tetracarboxyphthalocyanine

Mixture of trimellitic anhydride (10 g), urea (30 g), zinc (II) chloride (3.05 g) and ammonium molybdate (1 g) was agitated in nitrobenzene (200 ml) for 5 hours at 160-165°C. After self cooling precipitate was filtered and rinsed with water and acetone. Filter cake was stirred in the boiling 30% potassium hydroxide solution (200 ml) for 6 hours. After cooling reaction mixture was diluted with water (400 ml) and filtered. Filtrate was acidified with concentrated hydrochloric acid. Precipitate was filtered, suspended in water (200 ml), filtered and rinsed with water. Yield 0.78 g.

### EXAMPLE 5

Example 5 describes preparation of 9-(formyloxy)quinoxalino[2,3-b]phenazine-2-carboxylic acid, the predominantly planar polycyclic system of which is presented in Table 1, structural formula 44. The synthetic procedure is shown in Scheme 5 and comprises one step.

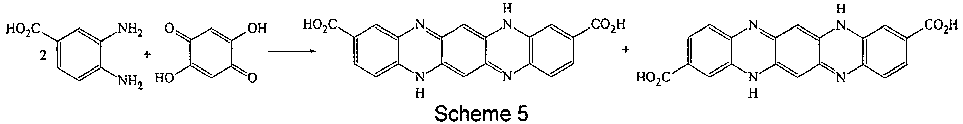

Mixture of 3,4-Diaminobenzoic acid (1.2 g) and 2,5-Dihydroxy-1,4-benzoquinone (0.5 g) in N-Methylpyrrolidone (30 ml) was boiled for 13 hours. Self cooled reaction mass was diluted with water (30 ml). Precipitate was filtered and rinsed with 50% N-Methylpyrrolidone and water. Filter cake was dissolved in the mixture of water (150 ml) and concentrated ammonia solution (10 ml). Acetic acid (10 ml) was added into the solution. Precipitate was filtered and rinsed with water on the filter. The product was isolated as a mixture of regioisomers.

### EXAMPLE 6

Example 6 describes preparation of N,N'-(4-octyl)phenyl-3,4,9,10-Perylenetetracarboxylic diimide, the predominantly planar polycyclic system of which is presented in Table 1, structural formula 19. This example is also representative for synthesis of compounds possessing polycyclic aromatic systems with structural formulas 1, 6, and 10 depicted in Table 1. The synthetic procedure is shown in Scheme 6 and comprises three steps.

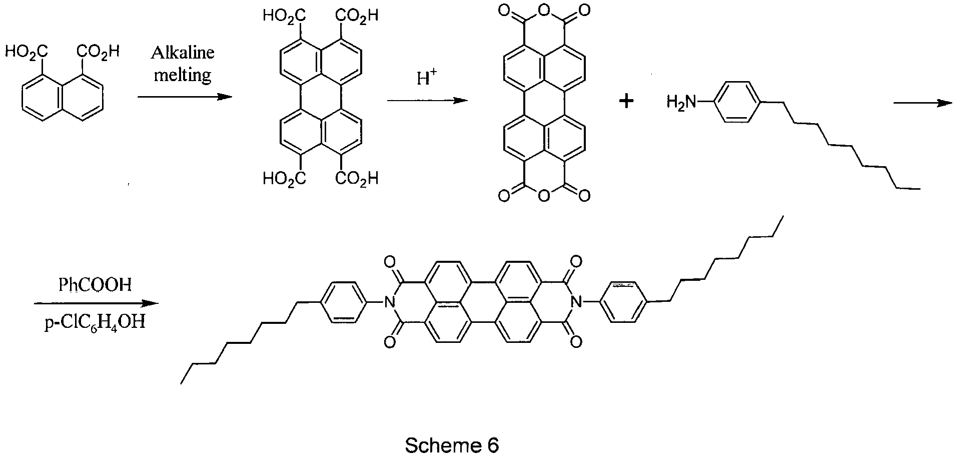

### A. Synthesis of perylene-3,4,9,10-tetracarboxylic acid dianhydride

Dianhydride of perylene-3,4,9,10-tetracarboxylic acid was prepared in two steps using standard procedure of alkaline melting to produce perylene-3,4,9,10-tetracarboxylic acid followed by acid-assisted dehydration leading to the corresponding dianhydride.

### B. Synthesis of N,N'-(4-octyl)phenyl-3,4,9,10-Perylenetetracarboxylic diimide

4-Octylaniline (2.10 g, 11 mmol), perylene-3,4,9,10-tetracarboxylic acid dianhydride (2.20 g, 3.70 mmol), and benzoic acid (0.020 g, 0.16 mmol) were mixed with m-CIPhOH was added (9 mL). The mixture was heated for 40 h at 150° C upon stirring. Solvent was removed *in vacuo,* residue was purified using column chromatography on silica gel using toluene-petroleum.ether mixture as eluent. Yield: 2.6 g (74%).

### EXAMPLE 7

Example 7 describes preparation of compounds A and B depicted in Scheme 7, the predominantly planar polycyclic systems of which is presented in Table 1, structural formulas 4 and 6. This example is also representative for synthesis of compounds possessing polycyclic aromatic systems with structural formula 7 depicted in Table 1. The synthetic route is shown in Scheme 7 and comprises one step starting from perylene-3,4,9,10-tetracarboxylic acid dianhydride, preparation of which is described in Example 6.

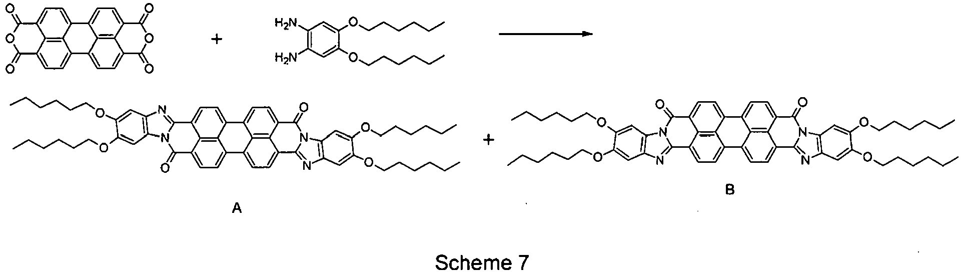

Compounds A and B were synthesized from perylene-3,4,9,10-tetracarboxylic acid dianhydride and 4,5-bis(hexyloxy)benzene-1,2-diamine using procedures similar to those described in Example 6. Typically, standard technique leads to the mixture of regio-isomers A and B which can be separated chromatographically.

### EXAMPLE 8

Example 8 describes preparation of compound C, the predominantly planar polycyclic system of which is presented in Table 1, structural formula 40. This example is also representative for synthesis of compounds possessing polycyclic aromatic systems with structural formula 39 depicted in Table 1. The synthetic procedure is shown in Scheme 8 and comprises one step. 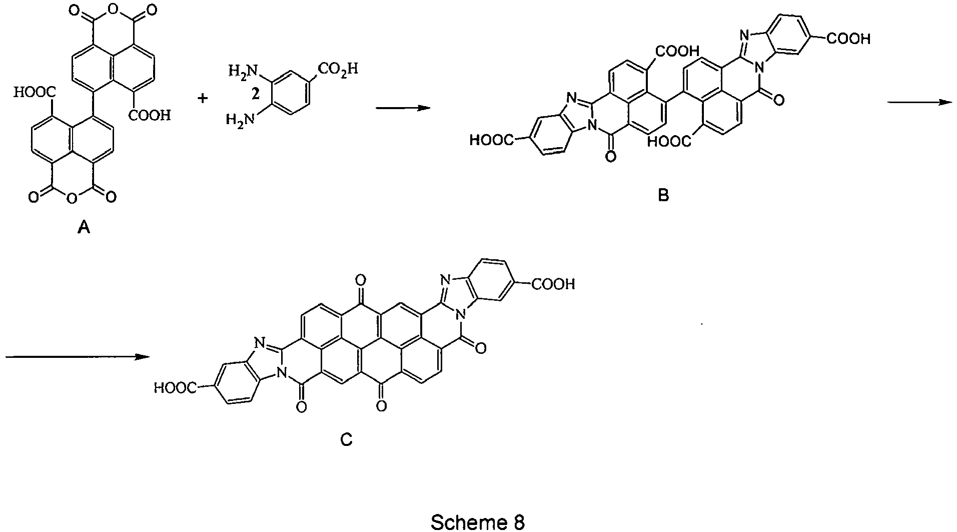 Compound C was synthesized from perylene1,1',3,3'-tetraoxo-1,1',3,3'-tetrahydro-6,6'-bibenzo[de]isochromene-7,7'-dicarboxylic acid and 3,4-diaminobenzoic acid using procedures similar to those described in Example 6. Typically, standard technique leads to the mixture of regio-isomers which can be separated chromatographically.

### EXAMPLE 9

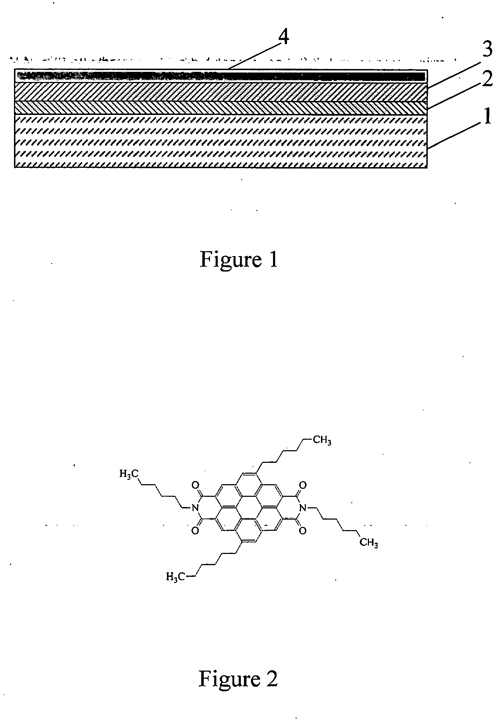

Dichroic polarizing element according to the present invention shown in Figure 1 is obtained via formation of at least one anisotropically absorbing optical layer 2 located on substrate 1. The layer 2 comprising oriented molecules of organic compound may be produced with one of the known methods.

Compound 10 is used for preparation of the polarizing element according to the present invention. Ethyl acetate is used as a solvent. The dyestuff was transformed into liquid crystal (LC) solution. The obtained LC solution is applied onto a glass plate (10x10 cm²) as a strip and on 1.5 cm distance from the edges of the substrate. The glass substrate is transparent for electromagnetic radiation in the visible spectral range. The substrate is fixed on the linearly moving stage. Non-rotating roller of 2-cm diameter is pressed against the substrate. The desired dyestuff solution layer thickness is controlled by two spacers fixed on the roller. The stage with the fastened substrate is moved with the speed of approximately 10 cm/sec. Orientation of the supramolecules of the LC solution is controlled by varying an external alignment force in the process of the layer formation. Different external alignment force may be used for this purpose, for example electro-magnetic, mechanical, et al. An intensity of the external alignment force is determined by properties of the LC solution: chemical content, concentration, temperature, etc. Layer thickness is approximately 5-10 µm.

Next step of the method is drying. It is required that the rate of solvent removal should not be too high in order to prevent disturbance of the previously oriented structure of the optically anisotropic organic layer. For the present experiment the drying was performed at room temperature and 60% humidity.

Then a transparent adhesive layer 3 (polyacrylate, polyvinylbutyral, etc.) was applied on top of the organic layer 2 according to the conventional manufacturing technology. To a material of an adhesive layer the following requirements (demands) were made: small absorption, a low parameter of refraction and insolubility in water.

At last a polymer protective layer 4 is formed for protecting the polarizing element from damage in the course of its transportation. In this example the polarizing element is a semi-product, which can be used as a polarizer, for example, in liquid crystal displays (LCDs). Upon the removal of protective layer 4, the remaining multi-layer structure is applied onto an LCD glass with adhesive layer.

The produced polarizing element is-comprises an optically anisotropic layer of approximately 0.3-0.4 µm thickness which possesses an average dichroic ratio equal to 7.0 (maximum is 9.0). The disclosed method has a good reproducibility of parameters both over the surface of the layer and from batch to batch.

### Example 10

Example 10 describes preparation of the polarizing element based on organic compound with the polycyclic system Y as shown in Table 1, structure 31. Controlling interactions between the molecules and therefore their self-assembling by choosing the chemical composition is the key factor tailoring the solubility. Amongst the possible interactions between polyaromatic molecules with alkyl periphery we can select pi-stacking and hydrophobic interactions as two main driving forces of lyomesophase formation.

Molecules with a pronounced tendency to self-associate are suitable for obtaining in-plane stacks distribution, since they possess the required pre-ordering. Tendency to aggregation depends on the chosen solvent. Aromatic solvents, like toluene, nitrobenzene, suppress pi-stacking between aromatic solutes as such interactions tend to prevail over interactions between solutes. In contrary, molecules of aliphatic solvent, like octane or decane, undergo hydrophobic interaction with molecular periphery improving the aromatic stacking. Coronene derivative as shown in Figure 2 is used for this example.

It is a flat polycyclic compound with alkyl substituents, soluble in a wide range of solvents. It gives lyomesophase at 20% solution in octane.

Lyotropic liquid crystal is deposited and an aligning shear force is applied. The coating liquid can be also prepared as isotropic solution. In this case there is an optimum relation between the rate of drying and the speed of deposition at which pre-aggregated species are aligned in the direction of deposition. Pre-treatment as rubbing of substrate also enhances the alignment.

The following films were deposited by rod at different speeds:
#1: MR 2.5, 5 mm/s (thickness 680 nm);
#2: MR 1.5, 20 mm/s (thickness 560 nm);
#3: MR 6.0, 100 mm/s (thickness 1770 nm).

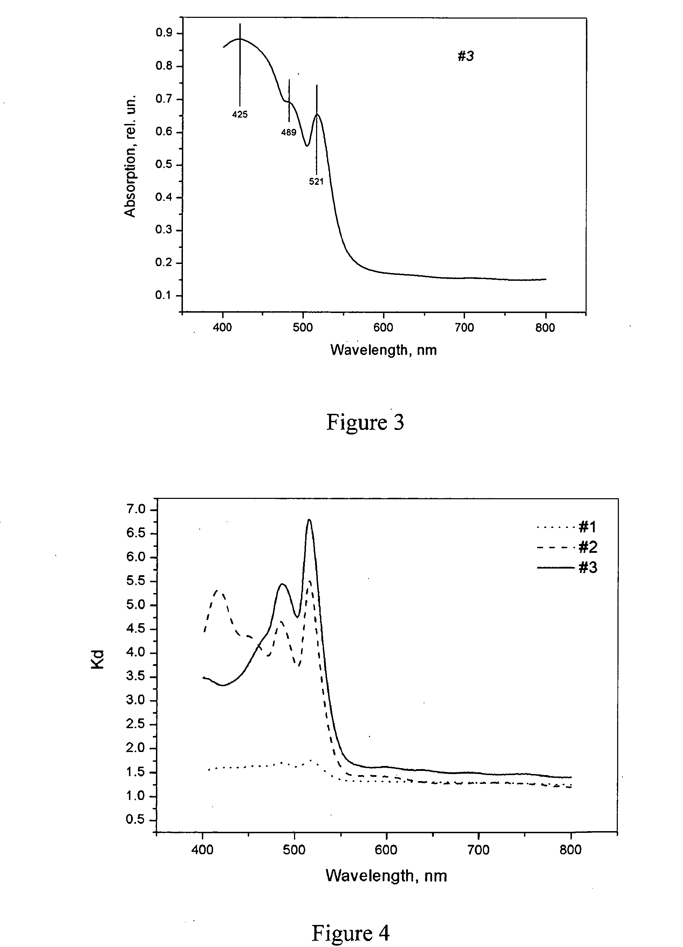

Figure 3 shows absorption spectrum of coronene film on glass. Three lines are clearly defined. Coronene has its lowest energy absorption band at 521 nm with two vibronic lines at 425 and 489 nm. At wavelengths higher than 521 nm the films are almost transparent.

Dichroic and contrast ratios of the samples are presented in Figures 3 and 4. Average Kd is 5.0 (maximum value is 7.0), whereas thickness-dependent CR reaches 40 for the thicker film. Present example demonstrates that we obtained polarizers of acceptable properties in blue spectral region.

While certain preferred embodiments of the invention have been specifically disclosed, it should be understood that the invention is not limited thereto as many variations will be readily apparent to those skilled in the art and the invention is to be given its broadest possible interpretation within the terms of the following claims.

## Claims

1. A polycyclic organic compound of a general structural formula I 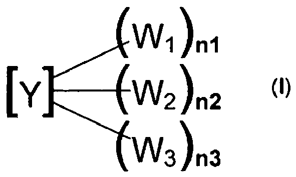
wherein Y is a predominantly planar polycyclic system being at least partially aromatic, W₁, W₂, and W₃ are different groups providing solubility in an organic solvent, and sum (n1+n2+n3) is 1, 2, 3, 4, 5, 6, 7, 8, 9 or 10,
wherein said polycyclic organic compound forms supramolecules in the organic solvent, and
said polycyclic organic compound is capable of absorbing electromagnetic radiation in at least one subrange of the visible spectral range,
wherein at least one of the W groups providing solubility is selected from the list comprising linear and branched (C₁-C₃₅)alkyl, (C₂-C₃₅)alkenyl, and (C₂-C₃₅)alkinyl groups,
wherein the polycyclic system Y comprises at least one fragment representing a polycyclic aromatic hydrocarbon, and
wherein the polycyclic aromatic hydrocarbon is selected from the list comprising acenaphthene, acenaphthylene, acephenanthrylene, aceanthrylene, anthanthrene, benzo[a]coronene, benzo[a]naphthacene, benzo[a]pyrene, benzo[b]chrysene, benzo[b]fluorene, benzo[c]chrysene, benzo[c]phenanthrene, benzo[e]pyrene, benzo[ghi]fluoranthene, benzo[ghi]naphtho[cde]perylene, benzo[ghi]perylene, benzo[j]fluoranthene, benzo[rst]dinaphtho[defg,ijkl]pentaphene, benzo[rst]phenanthro[1,10,9-cde]pentaphene, benz[a]anthracene, benz[e]acephenanthrylene, benz[rst]anthra[cde]pentaphene, biphenylene, chrysene, coronene, dibenzo[b,def]chrysene, dibenzo[bc,ef]coronene, dibenzo[cd,lm]perylene, dibenzo[g,p]chrysene, dibenzo[j,lm]naphtho[ab]perylene, dibenz[a,c]anthracene, dibenz[a,h]anthracene, dibenz[a,j]anthracene, dinaphtho[defg,opqr]pentacene, fluoranthene, fluorene, hexabenzo[a,cd,f,j,lm,o]perylene, naphthacene, naphthalene, naphtho[a]anthracene, naphtho[bcd]perylene, naphtho[d]coronene, pentabenzo[a,cd,f,j,lm]perylene, pentacene, pentaphene, perylene, phenanthrene, phenanthro[3,4-c]phenanthrene, picene, pyranthrene, pyrene, quaterrylene, tetrabenzo[a,cd,f,lm]perylene, terrylene, trinaphthylene, tetranaphthylene and triphenylene.

2. A polycyclic organic compound according to Claim 1, wherein the polycyclic system Y is heterocyclic, and wherein one or more heteroatoms of the heterocyclic system are selected from the list comprising N, O and S.

3. A polycyclic organic compound according to any of Claims from 1 to 2, wherein the polycyclic system Y comprises at least one fragment selected from the list comprising furan, oxirane, 4H-pyran, 2*H*-chromene, benzo[*b*]furan, 2*H*-pyran, thiophene, benzo[b]thiophene, parathiazine, pyrrole, pyrrolidine, pyrazole, imidazole, imidazoline, imidazolidine, pyrazolidine, pyrimidine, pyridine, piperazine, piperidine, pyrazine, indole, purine, benzimidazole, quinoline, phenothiazine, morpholine, thiaziole, thiadiazole, and oxazole.

4. A polycyclic organic compound according to any of Claims from 1 to 3, wherein the polycyclic system **Y** comprises fragments selected from the list comprising perylene, tetrapyrrolic macrocycles, coronene and pyrazine, and having a general structural formula selected from structures 1-46:
| | |
|---|---|
| 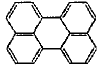 | 1 |
| 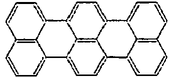 | 2 |
| 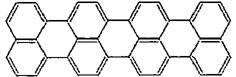 | 3 |
| 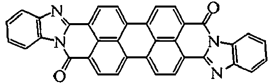 | 4 |
| 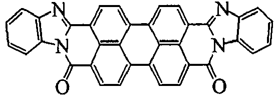 | 5 |
| 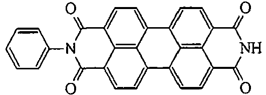 | 6 |
| 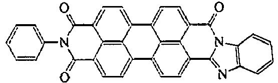 | 7 |
| 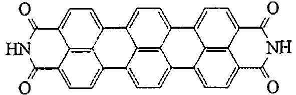 | 8 |
| 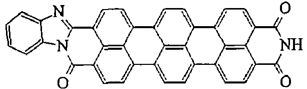 | 9 |
| 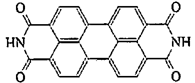 | 10 |
| 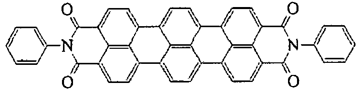 | 11 |
| 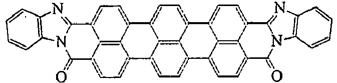 | 12 |
| 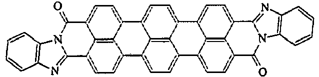 | 13 |
| 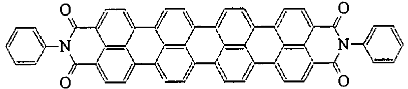 | 14 |
| 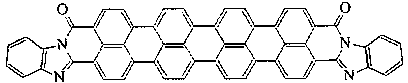 | 15 |
| 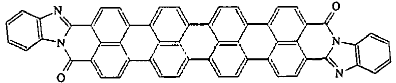 | 16 |
| 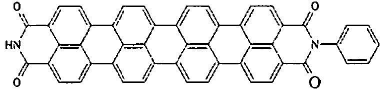 | 17 |
| 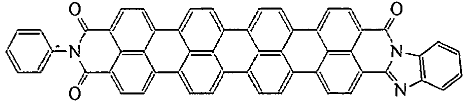 | 18 |
| 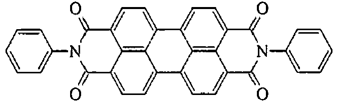 | 19 |
| 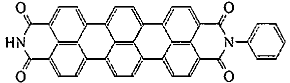 | 20 |
| 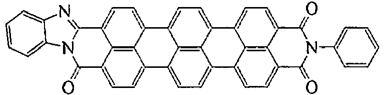 | 21 |
| 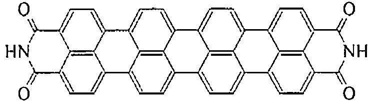 | 22 |
| 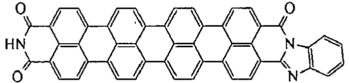 | 23 |
| 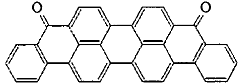 | 24 |
| 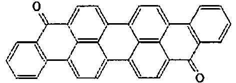 | 25 |
| 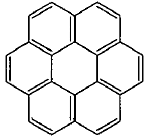 | 26 |
| 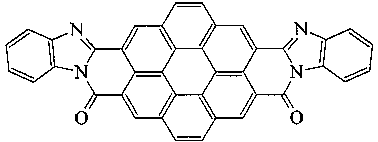 | 27 |
| 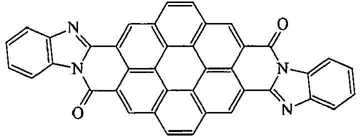 | 28 |
| 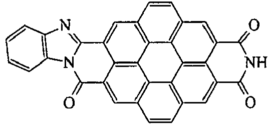 | 29 |
| 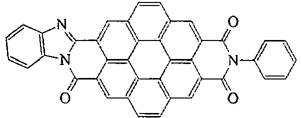 | 30 |
| 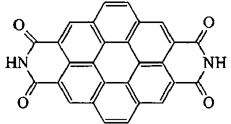 | 31 |
| 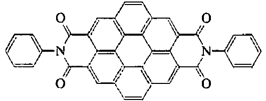 | 32 |
| 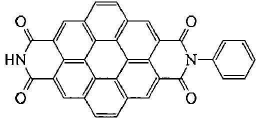 | 33 |
| 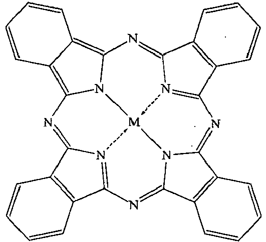 | 34 |
| 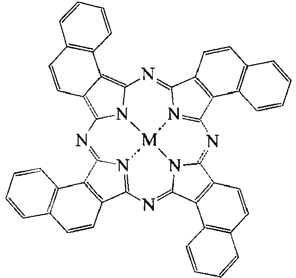 | 35 |
| 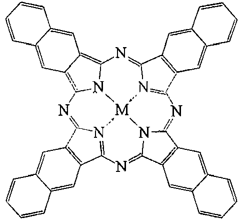 | 36 |
| 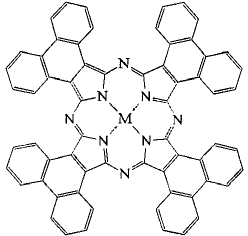 | 37 |
| 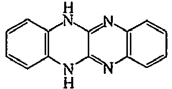 | 38 |
| 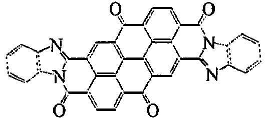 | 39 |
| 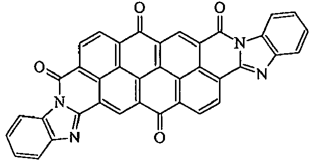 | 40 |
| 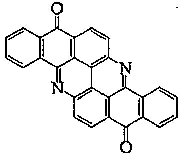 | 41 |
| 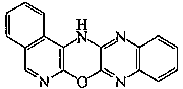 | 42 |
| 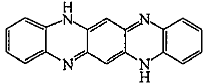 | 44 |
| 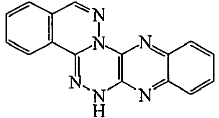 | 45 |
| 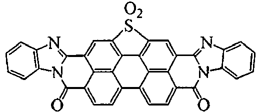 | 46 |
wherein **M** is selected from the list comprising 2H, Cu, Zn, Co, Fe and Pt

5. A polycyclic organic compound according to any of Claims from 1 to 4, wherein at least one of the **W** groups providing solubility is connected with the polycyclic system **Y** via a bridging group **A,** and wherein the bridging group **A** is selected from the list comprising -C(O)-, -C(O)O-, -C(O)-NH-, (SO₂)NH-, -O-, -CH₂O-, -NH-, >N-, and any combination thereof.

6. A polycyclic organic compound according to any of Claims 1, 4, or 5, selected from the list comprising diimides **I.1, I.2, I.3** and **I.4:** 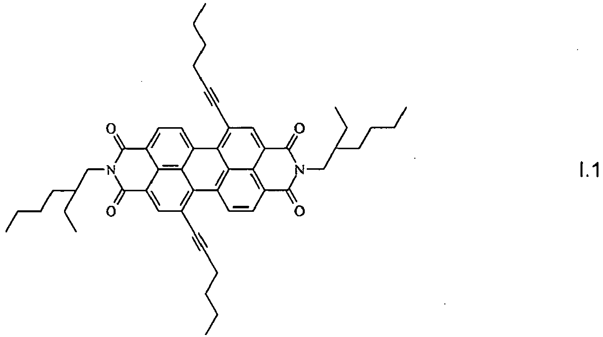 N,N'-di(2-ethylhexyl)-1,7-di(hex-1-ynyl)perylene-3,4:9,10-tetracarboxydiimide 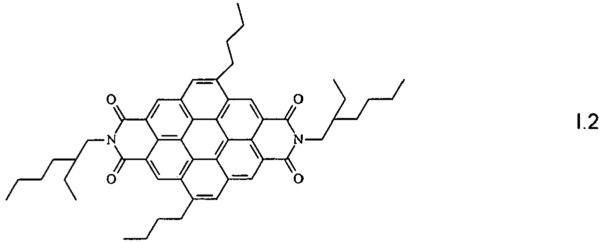 N,N'-di(2-ethylhexyl)-5,11-dibutylcoronene-2,3:8,9-tetracarboxydiimide 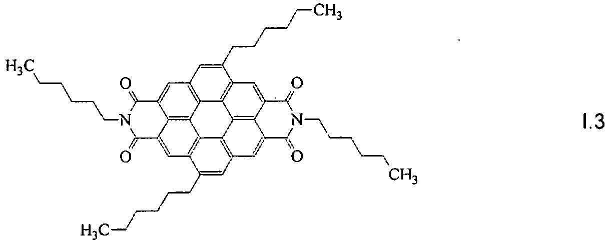 N,N'-dihexyl-5,11-dihexylcoronene-2,3:8,9-tetracarboxydiimide 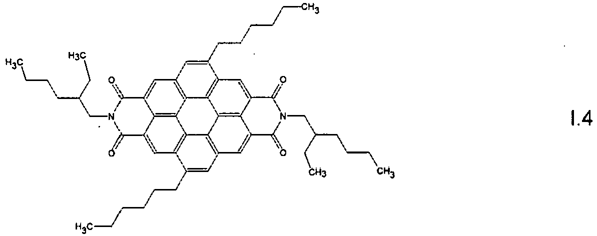 N,N'-di(2-ethylhexyl)-5,11-dihexylcoronene-2,3:8,9-tetracarboxydiimide

7. A polycyclic organic compound according to any of Claims from 1 to 6, wherein the polycyclic systems are capable of forming rod-like supramolecules via π-π-interaction.

8. A polycyclic organic compound according to Claim 7, wherein the rod-like supramolecules have interplanar spacing between the polycyclic systems in the range of approximately 3.1-3.7 A.

9. A polycyclic organic compound according to any of Claims from 1 to 8, wherein said compound is photochromic.

10. A polycyclic organic compound according to any of Claims from 1 to 9, wherein said polycyclic organic compound is further capable of absorbing electromagnetic radiation in at least one subrange of the UV spectral range.

11. A solution comprising at least one polycyclic organic compound according to any of Claims 1 to 10, wherein the solution is capable of forming an organic layer ensuring anisotropic absorption of electromagnetic radiation in at least one subrange of the visible spectral range.

12. A solution according to Claim 11, comprising a mix of at least two polycyclic organic compounds of a general structural formula I wherein said solution is capable of absorbing of electromagnetic radiation in at least two subranges of the visible spectral range.

13. A solution according to any of Claims from 11 to 12, wherein the organic solvent is selected from the list comprising ketones, carboxylic acids, hydrocarbons, cyclohydrocarbons, chlorohydrocarbons, alcohols, ethers, esters, acetone, xylene, toluene, ethanol, methylcyclohexane, ethyl acetate, diethyl ether, octane, chloroform, methylenechloride, dichloroethane, trichloroethene, tetrachloroethene, carbon tetrachloride, 1,4-dioxane, tetrahydrofuran, pyridine, triethylamine, nitromethane, acetonitrile, dimethylformamide, dimethulsulfoxide, and any combination thereof.

14. A solution according to any of Claims from 11 to 13, wherein the solution is a lyotropic liquid crystal solution or an isotropic solution.

15. A solution according to any of Claims from 11 to 14, wherein the supramolecules are formed by interaction of at least two different compounds of the general structural formula **I.**

16. A solution according to any of Claims from 11 to 15, wherein the supramolecules are formed by interaction of the same compounds of formula **I.**

17. A solution according to any of Claims from 11 to 16, further comprising surfactants or plasticizers.

18. A polarizing element, comprising at least one organic layer which is capable of anisotropic absorption of the electromagnetic radiation in at least one subrange of the visible spectral range, wherein the organic layer comprising at least one polycyclic organic compound, according to any of Claims 1 to 10.

19. A polarizing element according to Claim 18, wherein said layer comprises two or more said polycyclic compounds of the general structural formula I, ensuring absorption of electromagnetic radiation in at least two different predetermined wavelength subranges of the visible spectral range.

20. A polarizing element according to any of Claims from 18 to 19, comprising two or more organic layers, wherein each of said layers comprises different polycyclic compounds of the general structural formula **I** and ensuring the absorption of electromagnetic radiation in at least two predetermined wavelength subranges of the visible spectral range.

21. A polarizing element according to any of Claims 18 to 20, further comprising a substrate.

22. A polarizing element according to Claim 21, wherein the substrate is transparent for electromagnetic radiation in the visible spectral range.

23. A polarizing element according to any of Claims 21 or 22, wherein the substrate is made of polymer, glass or foil.

24. A polarizing element according to any of Claims from 18 to 23, further comprising a transparent adhesive layer applied on top of the organic layer.

25. A polarizing element according to Claim 24, further comprising a protective coating applied on the adhesive transparent layer.

26. A polarizing element according to any of Claims 18 to 25, wherein said optically anisotropic organic layer is at least partially crystalline.

27. A method of forming a polarizing element comprising the steps of:
preparation of a solution according to any of Claims 10 to 17,
deposition of a layer of the solution on a substrate, and
c) drying with formation of an optically anisotropic layer.

28. A method according to Claim 27, further comprising an application of an external orienting action on the solution layer to provide a dominant orientation of supramolecules, wherein the orienting action may take place after the step b) of the deposition of the solution layer or simultaneously with the step b), and wherein the orienting action is selected from the list comprising external mechanical, electromagnetic orienting actions and any combinations thereof.

29. A method according to any of Claims from 27 to 28, wherein the substrate is made of polymer, glass or foil.

30. A method according to any of Claims from 27 to 29, further comprising the step of removing the substrate by one of the methods from the list comprising wet chemical etching, dry chemical etching, plasma etching, laser etching, and grinding.

## Patentansprüche

1. Eine polyzyklische organische Verbindung mit einer allgemeinen Strukturformel I 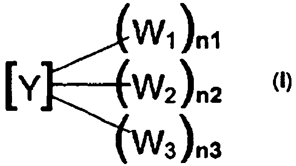
worin Y ein überwiegend planares polyzyklisches System ist, das zumindest zum Teil aromatisch ist, wobei W₁, W₂ und W₃ verschiedenen Gruppen sind, die Löslichkeit in einem organischen Lösungsmittel bereitstellen, und wobei die Summe (n1+n2+n3) 1, 2, 3, 4, 5, 6, 7, 8, 9 oder 10 ist,
worin die genannte polyzyklische organische Verbindung Supramoleküle in dem organischen Lösungsmittel bildet und die genannte polyzyklische organische Verbindung in der Lage ist, eine elektromagnetische Strahlung in mindestens einem Teilbereich des sichtbaren Spektralbereichs zu absorbieren,
worin mindestens eine der W-Gruppen, die die Löslichkeit bereitstellt, ausgewählt wird aus der Liste umfassend lineare und verzweigte (C₁-C₃₅)-Alkyl-, (C₂-C₃₅)-Alkenyl- und (C₂-C₃₅)-Alkinylgruppen,
worin das polyzyklische System Y mindestens ein Fragment umfasst, das einen polyzyklischen aromatischen Kohlenwasserstoff repräsentiert, und
worin der polyzyklische aromatische Kohlenwasserstoff ausgewählt wird aus der Liste umfassend Acenaphthen, Acenaphthylen, Acephenanthrylen, Aceanthrylen, Anthanthren, Benzo[a]coronen, Benzo[a]naphthacen, Benzo[a]pyren, Benzo[b]chrysen, Benzo[b]fluoren, Benzo[c]chrysen, Benzo[c]phenanthren, Benzo[e]pyren, Benzo[ghi]fluoranthen, Benzo[ghi]naphtho[cde]perylen, Benzo[ghi]perylen, Benzo[j]fluoranthen, Benzo[rst]dinaphtho[defg,ijkl]pentaphen, Benzo[rst]phenanthro[1,10,9-cde]pentaphen, Benz[a]anthracen, Benz[e]acephenanthrylen, Benz[rst]anthra[cde]pentaphen, Biphenylen, Chrysen, Coronen, Dibenzo[b,def]chrysen, Dibenzo[bc,ef]coronen, Dibenzo[cd,lm]perylen, Dibenzo[g,p]chrysen, Dibenzo(j,lm]naphtho[ab]perylen, Dibenz[a,c]anthracen, Dibenz[a,h]anthracen, Dibenz[aj]anthracen, Dinaphtho[defg,opqr]pentacen, Fluoranthen, Fluoren, Hexabenzo[a,cd,f,j,lm,o]perylen, Naphthacen, Naphthalen, Naphtho[a]anthracen, Naphtho[bcd]perylen, Naphtho[d]coronen, Pentabenzo[a,cd,f,j,lm]perylen, Pentacen, Pentaphen, Perylen, Phenanthren, Phenanthro[3,4-c]phenanthren, Picen, Pyranthren, Pyren, Quaterrylen, Tetrabenzo[a,cd,f,lm]perylen, Terrylen, Trinaphthylen, Tetranaphthylen und Triphenylen.

2. Eine polyzyklische organische Verbindung gemäß Anspruch 1, worin das polyzyklische System Y heterozyklisch ist und worin ein oder mehr Heteroatome des heterozyklischen Systems ausgewählt werden aus der Liste umfassend N, O und S.

3. Eine polyzyklische organische Verbindung gemäß einem der Ansprüche 1 bis 2, worin das polyzyklische System Y mindestens ein Fragment umfasst, das ausgewählt wird aus der Liste umfassend Furan, Oxiran, 4H-Pyran, 2H-Chromen, Benzo[b]furan, 2H-Pyran, Thiophen, Benzo[b]thiophen, Parathiazin, Pyrrol, Pyrrolidin, Pyrazol, Imidazol, Imidazolin, Imidazolidin, Pyrazolidin, Pyrimidin, Pyridin, Piperazin, Piperidin, Pyrazin, Indol, Purin, Benzimidazol, Chinolin, Phenothiazin, Morpholin, Thiazid, Thiadiazol und Oxazol.

4. Eine polyzyklische organische Verbindung gemäß einem der Ansprüche 1 bis 3, worin das polyzyklische System Y Fragmente umfasst, die ausgewählt werden aus der Liste umfassend Perylen, Tetrapyrrol-Makrozyklen, Coronen und Pyrazin, und die eine allgemeine Strukturformel hat, welche ausgewählt wird aus den Strukturen 1-46:
| | |
|---|---|
| 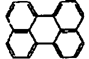 | 1 |
| 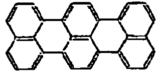 | 2 |
| 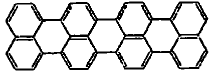 | 3 |
| 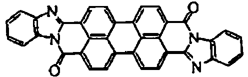 | 4 |
| 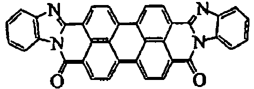 | 5 |
| 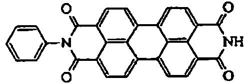 | 6 |
| 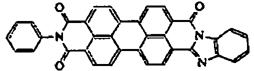 | 7 |
| 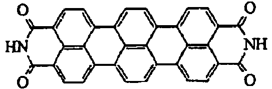 | 8 |
| 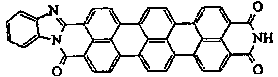 | 9 |
| 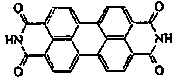 | 10 |
| 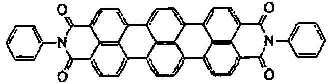 | 11 |
| 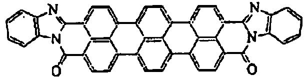 | 12 |
| 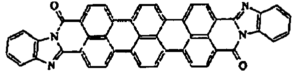 | 13 |
| 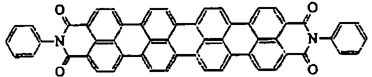 | 14 |
| 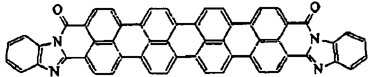 | 15 |
| 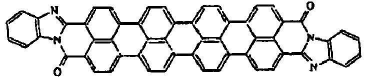 | 16 |
| 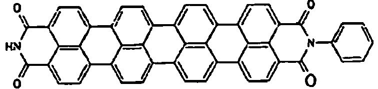 | 17 |
| 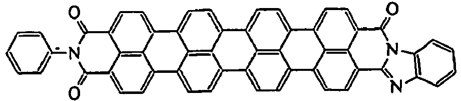 | 18 |
| 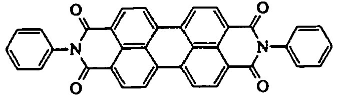 | 19 |
| 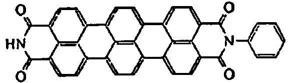 | 20 |
| 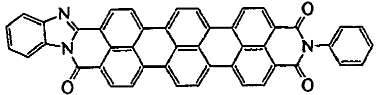 | 21 |
| 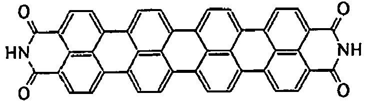 | 22 |
| 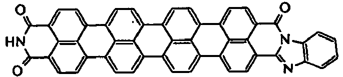 | 23 |
| 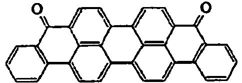 | 24 |
| 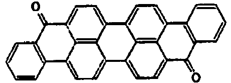 | 25 |
| 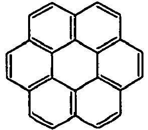 | 26 |
| 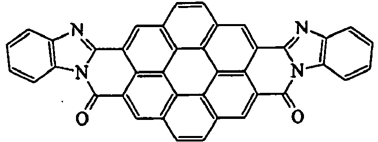 | 27 |
| 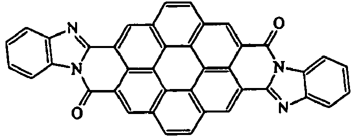 | 28 |
| 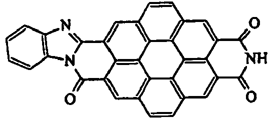 | 29 |
| 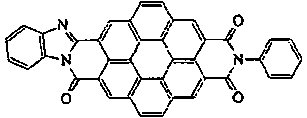 | 30 |
| 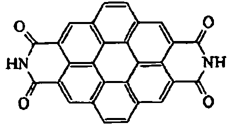 | 31 |
| 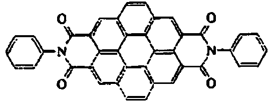 | 32 |
| 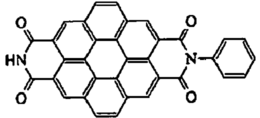 | 33 |
| 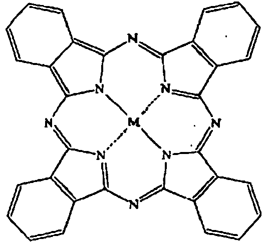 | 34 |
| 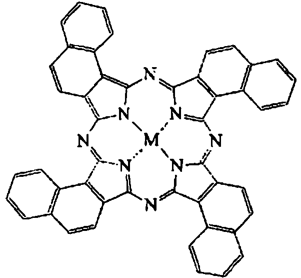 | 35 |
| 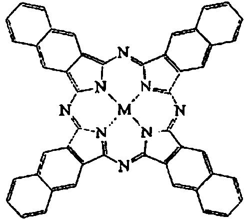 | 36 |
| 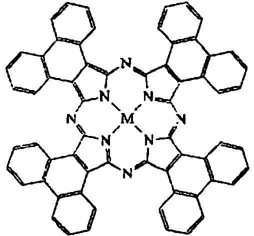 | 37 |
| 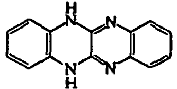 | 38 |
| 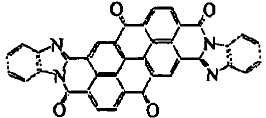 | 39 |
| 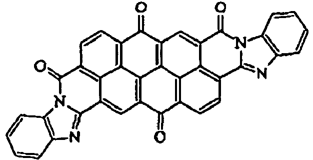 | 40 |
| 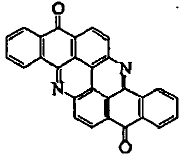 | 41 |
| 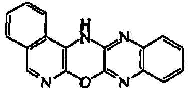 | 42 |
| 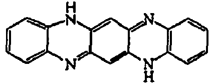 | 44 |
| 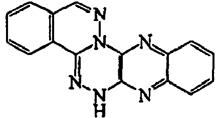 | 45 |
| 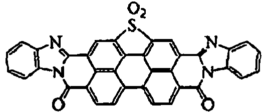 | 46 |
worin M ausgewählt wird aus der Liste umfassend 2H, Cu, Zn, Co, Fe und Pt

5. Eine polyzyklische organische Verbindung gemäß einem der Ansprüche 1 bis 4, worin mindestens eine der W-Gruppen, die die Löslichkeit bereitstellt, mit dem polyzyklischen System Y über eine überbrückende Gruppe A verbunden ist, und worin die überbrückende Gruppe A ausgewählt wird aus der Liste umfassend - C(O)-, -C(O)O- -C(O)-NH- -(SO₂)NH- -O-,-CH₂O- -NH-, >N- und eine Kombination davon.

6. Eine polyzyklische organische Verbindung gemäß einem der Ansprüche 1, 4 oder 5, die ausgewählt wird aus der Liste umfassend die Diimide I.1, I.2, I.3 und I.4: 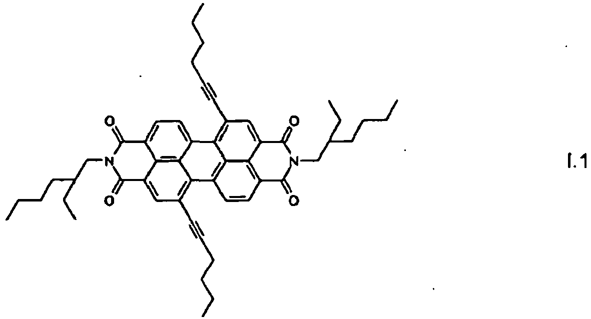 N,N'-Di(2-ethythexyl)-1,7-di(hex-1-ynyl)perylen-3,4:9,10-tetracarboxydiimid 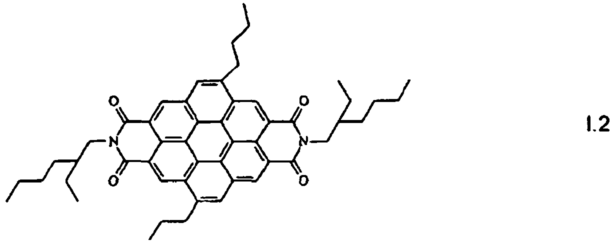 N,N'-Di(2-ethylhexyl)-5,11-butylcoronen-2,3:8,9-tetracarboxydiimid 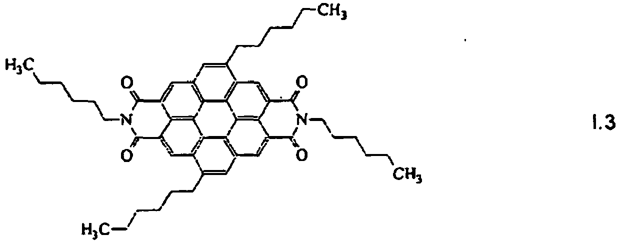 N,N'-Dihexyl-5,11-dihexylcoronen-2,3:8,9-tetracarboxydiimid 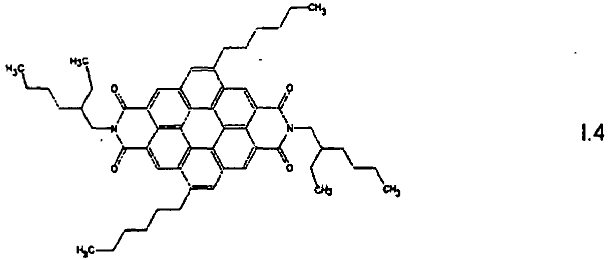 N,N'-Di(2-ethylhexyl)-5,11-dihexylcoronen-2,3:8,9-tetracarboxydiimid

7. Eine polyzyklische organische Verbindung gemäß einem der Ansprüche 1 bis 8, worin die genannte Verbindung photochrom ist.

8. Eine polyzyklische organische Verbindung gemäß einem der Ansprüche 1 bis 9, worin die genannte polyzyklische organische Verbindung überdies in der Lage ist, elektromagnetische Strahlung in mindestens einem Teilbereich des sichtbaren UV-Spektralbereichs zu absorbieren.

9. Eine polyzyklische organische Verbindung gemäß einem der Ansprüche 1 bis 6, worin die polyzyklischen Systeme in der Lage sind, stabförmige Supramoleküle über eine π-π-Interaktion zu bilden.

10. Eine polyzyklische organische Verbindung gemäß Anspruch 7, worin die stabförmigen Supramoleküle einen interplanaren Abstand zwischen den polyzyklischen Systemen im Bereich von ca. 3.1-3.7 A aufweisen.

11. Eine Lösung umfassend mindestens eine polyzyklische organische Verbindung gemäß einem der Ansprüche 1 bis 10, worin die Lösung in der Lage ist, eine organische Schicht zu bilden, welche die anisotrope Absorption von elektromagnetischer Strahlung in mindestens einem Teilbereich des sichtbaren Spektralbereichs sicherstellt.

12. Eine Lösung gemäß Anspruch 11, umfassend eine Mischung von mindestens zwei polyzyklischen organischen Verbindungen mit einer allgemeinen Strukturformel I, worin die genannte Lösung in der Lage ist, elektromagnetische Strahlung in mindestens zwei Teilbereichen des sichtbaren Spektralbereichs zu absorbieren.

13. Eine Lösung gemäß einem der Ansprüche 11 bis 12, worin das organische Lösungsmittel ausgewählt wird aus der Liste umfassend Ketone, Carboxylsäuren, hydrierte Kohlenwasserstoffe, zyklische Kohlenwasserstoffe, chlorierte Kohlenwasserstoffe, Alkohole, Ether, Ester, Aceton, Xylol, Toluol, Ethanol, Methylcyclohexan, Ethylacetat, Diethylether, Octan, Chloroform, Methylenchlorid, Dichlorethan, Trichlorethen, Tetrachlorethen, Tetrachlorkohlenstoff, 1,4-Dioxan, Tetrahydrofuran, Pyridin, Triethylamin, Nitromethan, Acetonitril, Dimethylformamid, Dimethulsulfoxid und Kombinationen davon.

14. Eine Lösung gemäß einem der Ansprüche 11 bis 13, worin die Lösung eine lyotrope Flüssigkristalllösung oder eine isotrope Lösung ist.

15. Eine Lösung gemäß einem der Ansprüche 11 bis 14, worin die Supramoleküle durch Interaktion von mindestens zwei verschiedenen Verbindungen mit der allgemeinen Strukturformel I gebildet werden.

16. Eine Lösung gemäß einem der Ansprüche 11 bis 15, worin die Supramoleküle durch Interaktion derselben Verbindungen mit der Formel I gebildet werden.

17. Eine Lösung gemäß einem der Ansprüche 11 bis 16, überdies umfassend Tenside oder Weichmacher.

18. Ein polarisierendes Element, umfassend mindestens eine organische Schicht, die fähig ist zur anisotropen Absorption der elektromagnetischen Strahlung in mindestens einem Teilbereich des sichtbaren Spektralbereichs, worin die organische Schicht mindestens eine polyzyklische organische Verbindung gemäß einem der Ansprüche 1 bis 10 umfasst.

19. Ein polarisierendes Element gemäß Anspruch 18, worin die genannte Schicht zwei oder mehr polyzyklische Verbindungen mit der allgemeinen Strukturformel I umfasst, wodurch die Absorption von elektromagnetischer Strahlung in mindestens zwei verschiedenen vorher festgelegten Wellenlängen-Teilbereichen des sichtbaren Spektralbereichs gewährleistet ist.

20. Ein polarisierendes Element gemäß einem der Ansprüche 18 bis 19, das zwei oder mehr organische Schichten umfasst, worin jede der Schichten verschiedene polyzyklische Verbindungen mit der allgemeinen Strukturformel I umfasst, und wodurch die Absorption von elektromagnetischer Strahlung in mindestens zwei verschiedenen vorher festgelegten Wellenlängen-Teilbereichen des sichtbaren Spektralbereichs sichergestellt wird.

21. Ein polarisierendes Element gemäß einem der Ansprüche 18 bis 20, das überdies ein Substrat umfasst.

22. Ein polarisierendes Element gemäß Anspruch 21, worin das Substrat für elektromagnetische Strahlung im sichtbaren Spektralbereich durchlässig ist.

23. Ein polarisierendes Element gemäß einem der Ansprüche 21 bis 22, worin das Substrat aus Polymer, Glas oder Folie besteht.

24. Ein polarisierendes Element gemäß einem der Ansprüche 18 bis 23, das überdies eine transparente Klebeschicht, die auf die organische Schicht aufgetragen wird, umfasst.

25. Ein polarisierendes Element gemäß Anspruch 24, das überdies eine Schutzschicht, die auf die transparente Klebeschicht aufgetragen wird, umfasst.

26. Ein polarisierendes Element gemäß einem der Ansprüche 18 bis 26, worin die genannte optisch anisotrope organische Schicht zumindest teilweise kristallin ist.

27. Ein Verfahren zur Bildung eines polarisierenden Elements, umfassend die Schritte:
Herstellung einer Lösung gemäß einem der Ansprüche 10 bis 17, Abscheidung einer Schicht der Lösung auf ein Substrat und c) Trocknen unter Bildung einer optisch anisotropen Schicht.

28. Ein Verfahren gemäß Anspruch 27, überdies umfassend das Anwenden eines externen orientierenden Vorgangs auf der Lösungsschicht, um eine dominante Orientierung der Supramoleküle bereitzustellen, worin der orientierende Vorgang nach dem Schritt b) der Abscheidung der Lösungsschicht oder gleichzeitig mit dem Schritt b) stattfinden kann, und worin der Orientierungsvorgang ausgewählt wird aus der Liste umfassend externe mechanische, elektromagnetische Orientierungsvorgänge und Kombinationen davon.

29. Ein Verfahren gemäß einem der Ansprüche 27 bis 28, worin das Substrat aus Polymer, Glas oder Folie besteht.

30. Ein Verfahren gemäß einem der Ansprüche 27 bis 29, überdies umfassend den Schritt des Entfernens des Substrats mittels eines dieser Verfahren von der Liste umfassend nasschemisches Ätzen, trockenchemisches Ätzen, Plasmaätzen, Laserätzen und Schleifen.

## Revendications

1. Un composé organique polycyclique de la formule de structure générale I 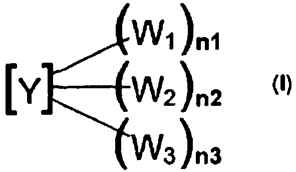
où Y est un système polycyclique principalement planaire qui est au moins partiellement aromatique,
W₁, W₂ et W₃ sont des groupes différents fournissant une solubilité dans un solvant organique et la somme (n1+n2+n3) est 1, 2, 3, 4, 5, 6, 7, 8, 9 ou 10,
où ledit composé organique polycyclique forme des supramolécules dans le solvant organique et ledit composé organique polycyclique est capable d'absorber un rayonnement électromagnétique dans au moins un sous-domaine du domaine spectral visible,
où au moins un des groupes W fournissant une solubilité est sélectionné dans la liste comprenant des groupes linéaires et ramifiés (C₁-C₃₅)alkyle, (C₂-C₃₅)alcényle et (C₂-C₃₅)alkinyle,
où le système polycyclique Y comprend au moins un fragment représentant un hydrocarbure aromatique polycyclique, et
où l'hydrocarbure aromatique polycyclique est sélectionné dans la liste comprenant acénaphthène, acénaphthylène, acéphénanthrylène, acéanthrylène, anthanthrène, benzo[a]coronène, benzo[a]naphthacène, benzo[a]pyrène, benzo[b]chrysène, benzo[b]fluorène, benzo[c]chrysène, benzo[c]phénanthrène, benzo[e]pyrène, benzo[ghi]fluoranthène, benzo[ghi]naphtho[cde]pérylène, benzo[ghi]pérylène, benzo[j]fluoranthène, benzo[rst]dinaphtho[defg,ijkl]pentaphène, benzo[rst]phénanthro[1,10,9-cde]pentaphène, benz[a]anthracène, benz[e]acéphénanthrylène, benz[rst]anthra[cde]pentaphène, biphénylène, chrysène, coronène, dibenzo[b,def]chrysène, dibenzo[bc,ef]coronène, dibenzo[cd,lm]pérylène, dibenzo[g,p]chrysène, dibenzo(j,lm]naphtho[ab]pérylène, dibenz[a,c]anthracène, dibenz[a,h]anthracène, dibenz[aj]anthracène, dinaphtho[defg,opqr]pentacène, fluoranthène, fluorène, hexabenzo[a,cd,f,j,lm,o]pérylène, naphthacène, naphthalène, naphtho[a]anthracène, naphtho[bcd]pérylène, naphtho[d]coronène, pentabenzo[a,cd,f,j,lm]pérylène, pentacène, pentaphène, pérylène, phénanthrène, phénanthro[3,4-c]phénanthrène, picène, pyranthrène, pyrène, quaterrylène, tétrabenzo[a,cd,f,lm]pérylène, terrylène, trinaphthylène, tétranaphthylène et triphénylène.

2. Un composé organique polycyclique selon la Revendication 1, où le système polycyclique Y est hétérocyclique, et où un ou plusieurs hétéroatomes du système hétérocyclique sont sélectionnés dans la liste comprenant N, O et S.

3. Un composé organique polycyclique selon l'une quelconque des Revendications 1 à 2, où le système polycyclique Y comprend au moins un fragment sélectionné dans la liste comprenant furane, oxyrane, 4H-pyrane, 2*H*-chromène, benzo[*b*]furane, 2H-pyrane, thiophène, benzo[*b*]thiophène, parathiazine, pyrrole, pyrrolidine, pyrazole, imidazole, imidazoline, imidazolidine, pyrazolidine, pyrimidine, pyridine, pipérazine, pipéridine, pyrazine, indole, purine, benzimidazole, quinoline, phénothiazine, morpholine, thiazide, thiadiazole et oxazole.

4. Un composé organique polycyclique selon l'une quelconque des Revendications 1 à 3, où le système polycyclique Y comprend des fragments sélectionnés dans la liste comprenant pérylène, macrocycles tétrapyrroliques, coronène et pyrazine et possède une formule de structure générale sélectionnée parmi les structures 1 à 46 :
| | |
|---|---|
| 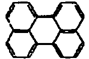 | 1 |
| 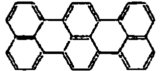 | 2 |
| 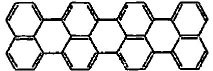 | 3 |
| 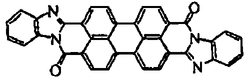 | 4 |
| 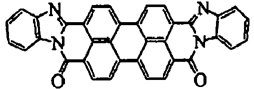 | 5 |
| 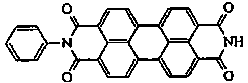 | 6 |
| 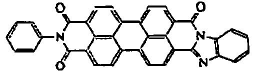 | 7 |
| 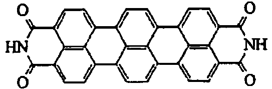 | 8 |
| 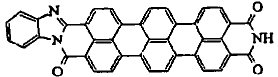 | 9 |
| 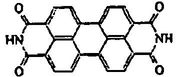 | 10 |
| 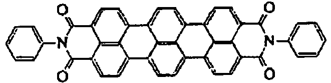 | 11 |
| 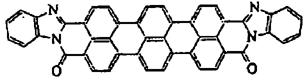 | 12 |
| 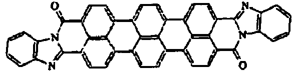 | 13 |
| 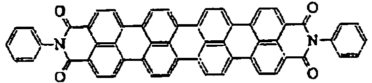 | 14 |
| 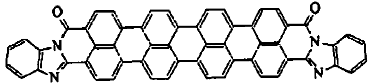 | 15 |
| 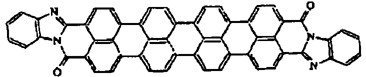 | 16 |
| 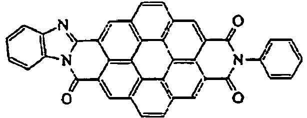 | 30 |
| 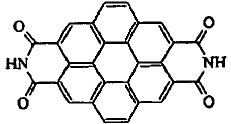 | 31 |
| 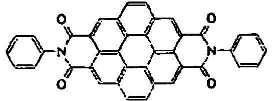 | 32 |
| 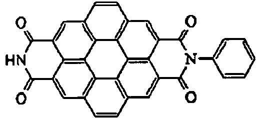 | 33 |
| 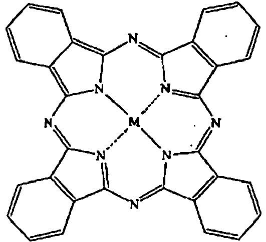 | 34 |
| 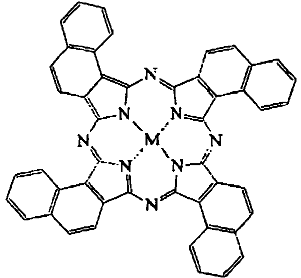 | 35 |
| 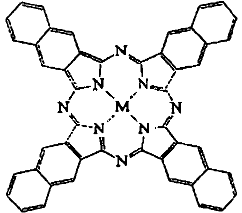 | 36 |
| 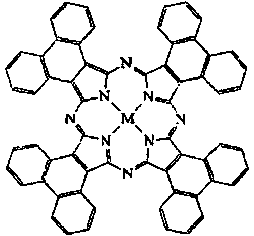 | 37 |
| 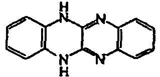 | 38 |
| 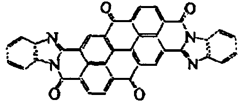 | 39 |
| 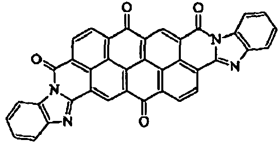 | 40 |
| 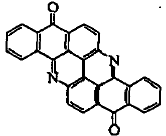 | 41 |
| 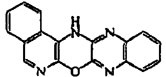 | 42 |
| 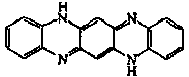 | 44 |
| 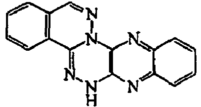 | 45 |
| 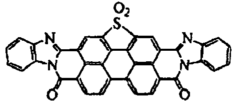 | 46 |
où M est sélectionné dans la liste comprenant 2H, Cu, Zn, Co, Fe et Pt.

5. Un composé organique polycyclique selon l'une quelconque des Revendications 1 à 4, où au moins un des groupes W fournissant une solubilité est raccordé au système polycyclique Y par l'intermédiaire d'un groupe en pont A, et où le groupe en pont A est sélectionné dans la liste comprenant -C(O)-, - C(O)O-, -C(O)-NH-, -(SO₂)NH-, -O-,-CH₂O-, -NH-, >N-, et toute combinaison de ceux-ci.

6. Un composé organique polycyclique selon l'une quelconque des Revendications 1, 4 ou 5, sélectionné dans la liste comprenant diimides 1.1, I.2, I.3 et I.4 : 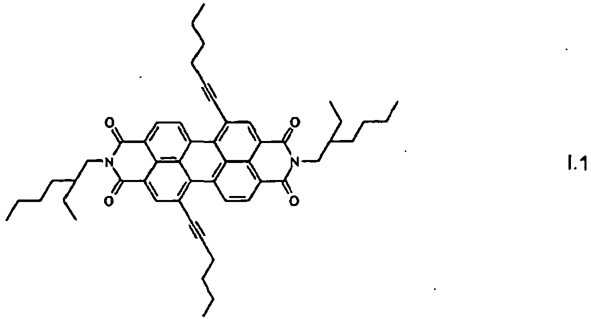 N,N'-di(2-éthyhexyle)-1,7-di(hex-1-ynyl)pérylène-3,4:9,10-tétracarboxydiimide 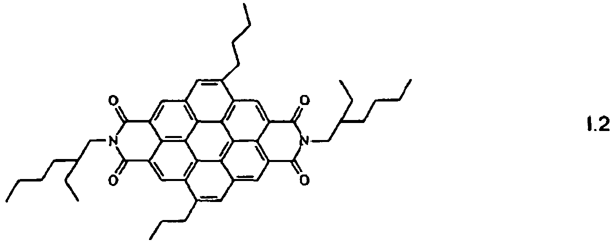 N,N'-di(2-éthylhexyl)-5,11-dibutylcoronène-2,3:8,9-tétracarboxydiimide 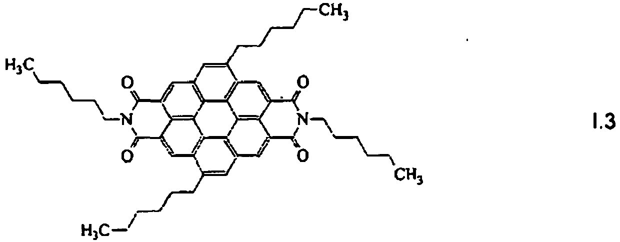 N,N'-dihexyl-5,11-dihexylcoronène-2,3:8,9-tétracarboxydiimide 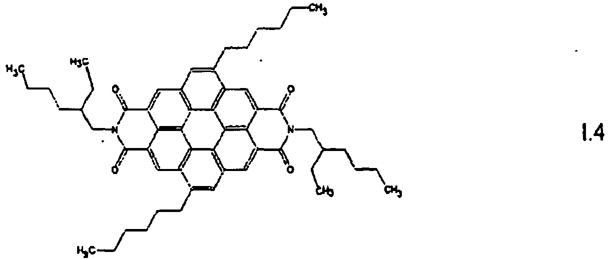 N,N'-di(2-éthylhexyl)-5,11-dihexylcoronène-2,3:8,9-tétracarboxydiimide

7. Un composé organique polycyclique selon l'une quelconque des Revendications 1 à 6, où les systèmes polycycliques sont capables de former des supramolécules en forme de tige par l'intermédiaire d'une interaction π-π.

8. Un composé organique polycyclique selon la Revendication 7, où les supramolécules en forme de tige possèdent un espacement interplanaire entre les systèmes polycycliques dans la plage d'approximativement 3,1 à 3,7 A.

9. Un composé organique polycyclique selon l'une quelconque des Revendications 1 à 8, où ledit composé est photochromique.

10. Un composé organique polycyclique selon l'une quelconque des Revendications 1 à 9, où ledit composé organique polycyclique est en outre capable d'absorber un rayonnement électromagnétique dans au moins un sous-domaine du domaine spectral UV.

11. Une solution contenant au moins un composé organique polycyclique selon l'une quelconque des Revendications 1 à 10, où la solution est capable de former une couche organique garantissant une absorption anisotrope d'un rayonnement électromagnétique dans au moins un sous-domaine du domaine spectral visible.

12. Une solution selon la Revendication 11, comprenant un mélange d'au moins deux composés organiques polycycliques de la formule de structure générale I, où ladite solution est capable d'absorber un rayonnement électromagnétique dans au moins deux sous-domaines du domaine spectral visible.

13. Une solution selon l'une quelconque des Revendications 11 à 12, où le solvant organique est sélectionné dans la liste comprenant cétones, acides carboxyliques, hydrocarbures, cyclohydrocarbures, chlorohydrocarbures, alcools, éthers, esters, acétone, xylène, toluène, éthanol, méthylcyclohexane, acétate d'éthyle, éther diéthylique, octane, chloroforme, chlorure de méthylène, dichloroéthane, trichloroéthène, tétrachloroéthène, tétrachlorure de carbone, 1,4-dioxane, tétrahydrofurane, pyridine, triéthylamine, nitrométhane, acétonitrile, diméthylformamide, diméthulsulfoxyde, et toute combinaison de ceux-ci.

14. Une solution selon l'une quelconque des Revendications 11 à 13, où la solution est une solution de cristaux liquides lyotropes ou une solution isotrope.

15. Une solution selon l'une quelconque des Revendications 11 à 14, où les supramolécules sont formées par une interaction d'au moins deux composés différents de la formule de structure générale I.

16. Une solution selon l'une quelconque des Revendications 11 à 15, où les supramolécules sont formées par une interaction des mêmes composés que ceux de la formule I.

17. Une solution selon l'une quelconque des Revendications 11 à 16, contenant en outre des agents tensioactifs ou des plastifiants.

18. Un élément de polarisation, comprenant au moins une couche organique qui est capable d'une absorption anisotrope du rayonnement électromagnétique dans au moins un sous-domaine du domaine spectral visible, où la couche organique comprend au moins un composé organique polycyclique selon l'une quelconque des Revendications 1 à 10.

19. Un élément de polarisation selon la Revendication 18, où ladite couche comprend deux ou plus desdits composés polycycliques de la formule de structure générale I, garantissant une absorption d'un rayonnement électromagnétique dans au moins deux sous-domaines de longueurs d'onde prédéterminées différentes du domaine spectral visible.

20. Un élément de polarisation selon l'une quelconque des Revendications 18 à 19, comprenant deux ou plus couche organiques, où chacune desdites couches comprend des composés polycycliques différents de la formule de structure générale I et qui garantissent l'absorption d'un rayonnement électromagnétique dans au moins deux sous-domaines de longueurs d'onde prédéterminées différentes du domaine spectral visible.

21. Un élément de polarisation selon l'une quelconque des Revendications 18 à 20, comprenant en outre un substrat.

22. Un élément de polarisation selon la Revendication 21, où le substrat est transparent pour un rayonnement électromagnétique dans le domaine spectral visible.

23. Un élément de polarisation selon l'une quelconque des Revendications 21 ou 22, où le substrat est constitué de polymère, verre ou feuille.

24. Un élément de polarisation selon l'une quelconque des Revendications 18 à 23, comprenant en outre une couche adhésive transparente appliquée au-dessus de la couche organique.

25. Un élément de polarisation selon la Revendication 24, comprenant en outre un revêtement protecteur appliqué sur la couche adhésive transparente.

26. Un élément de polarisation selon l'une quelconque des Revendications 18 à 25, où ladite couche organique optiquement anisotrope est au moins partiellement cristalline.

27. Un procédé de formation d'un élément de polarisation comprenant les opérations suivantes :
la préparation d'une solution selon l'une quelconque des Revendications 10 à 17,
le dépôt d'une couche de la solution sur un substrat, et
c) le séchage avec la formation d'une couche optiquement anisotrope.

28. Un procédé selon la Revendication 27, comprenant en outre une application d'une action d'orientation externe sur la couche de solution de façon à fournir une orientation dominante de supramolécules, où l'action d'orientation peut se dérouler après l'opération b) de dépôt de la couche de solution ou simultanément avec l'opération b), et où l'action d'orientation est sélectionnée dans la liste comprenant des actions d'orientation électromagnétique, mécanique externe, et toutes combinaisons de celles-ci.

29. Un procédé selon l'une quelconque des Revendications 27 à 28, où le substrat est constitué de polymère, verre ou feuille.

30. Un procédé selon l'une quelconque des Revendications 27 à 29, comprenant en outre l'opération de retrait du substrat par l'un des procédés de la liste comprenant gravure chimique humide, gravure chimique sèche, gravure au plasma, gravure laser et meulage.
